# EUROPÄISCHE PATENTANMELDUNG

(11) **EP 0 261 459 A2**
(43) Veröffentlichungstag der Anmeldung: **30.03.1988**
(21) Anmeldenummer: 87112784.1
(22) Anmeldetag: 02.09.1987
(51) Int. Cl.: C07D 277/82, C07D 417/12, C07D 513/04, A01N 43/80, A01N 43/90, A01N 47/34

(54) **Neue N-Benzothiazolylamide**

(30) Priorität: 09.09.1986 JP 210760/86; 17.03.1987 JP 60129/87
(71) Anmelder: NIHON TOKUSHU NOYAKU SEIZO K.K., Chuo-ku Tokyo 103 (JP)
(72) Erfinder: Kume, Toyohiko, Hino-shi Tokyo (JP); Tsuboi, Shinichi, Hino-shi Tokyo (JP); Sasaki, Shoko, Hino-shi Tokyo (JP); Yanagi, Akihiko, Oume-shi Tokyo (JP); Hattori Yumi, Hachioji-shi Tokyo (JP); Yagi, Shigeki, Tokyo (JP); Sirrenberg, Wilhelm, Dr., D-4322 Sprockhoevel (DE); Becker, Benedikt, Dr., D-4020 Mettmann (DE)
(74) Vertreter: Schumacher, Günter, Dr.

(57) **Zusammenfassung**

Offenbart werden neue N-Benzothiazolylamide der Formel (I) 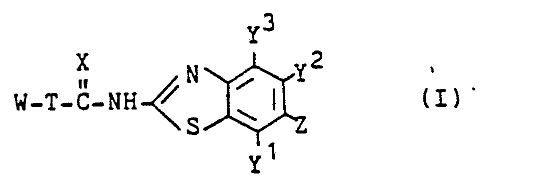
und die Verwendung der neuen Verbindungen als Insektizide.

## Beschreibung

Die vorliegende Erfindung betrifft neue N-Benzothiazolylamide, Verfahren zu ihrer Herstellung und ihre Verwendung als Insektizide.

Es wurde bereits offenbart, daß bestimmte Benzoylharnstoffe insektizide Aktivitäten aufweisen (siehe die JP-OS 124 265/1978) und daß ein bestimmtes Benzamid ebenfalls insektizide Aktivitäten besitzt (siehe J. Agric. Food Chem. Band 24, 1976, Seiten 1065-1068).

Nunmehr wurden neue N-Benzothiazolylamide der Formel (I) 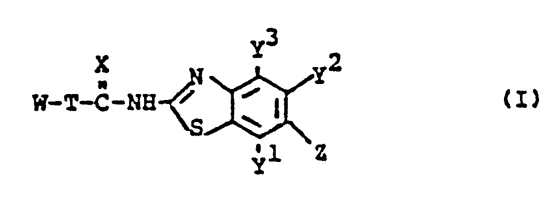
gefunden, in der
X Sauerstoff oder Schwefel ist,
T eine Einfachbindung oder -CONH-ist, worin der Pfeil "-" eine Bindung mit W bezeichnet, Y¹, Y² und Y³ gleich oder verschieden sind und H, Halogen oder Alkyl bezeichnen,
Z Halogen, Alkoxy, Halogenoalkoxy, Aralkyloxy, Alkenyloxy, Halogenoalkenyloxy, Alkinyloxy,

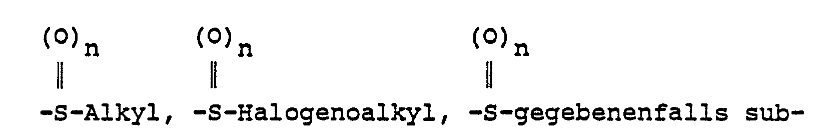

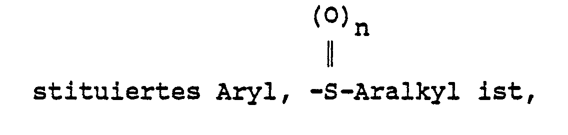

worin n jeweils 0, 1 oder 2 ist, oder

Z Aryl, gegebenenfalls substituiertes Aryloxy. -O-Q ist, worin Q eine gegebenenfalls substituierte 5-bis 6- gliedrige heterocyclische Gruppe ist, oder 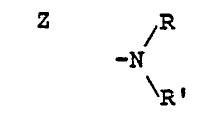 ist, worin R und R' gleich oder verschieden sind und Alkyl oder Alkenyl bezeichnen und R und R' zusammen mit dem N-Atom, an das sie gebunden sind, eine gegebenenfalls substituierte 5-bis 6-gliedrige heterocyclische Gruppe bilden können, die Hetero-Atome neben einem Stickstoff-Atom enthalten kann, oder

Z zusammen mit Y¹ oder Y² eine Tetrafluoroethylendioxy-Gruppe bilden kann und 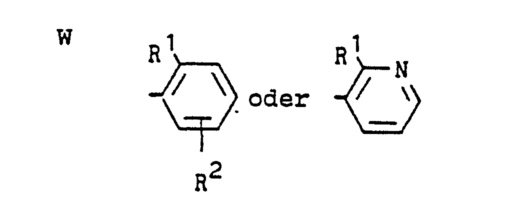 ist, worin R¹ Halogen, Alkyl oder Alkoxy ist und R² Wasserstoff, Halogen, Alkyl oder Alkoxy ist, mit der Maßgabe, daß
(a) in dem Fall, in dem T -CONH-ist, einer oder zwei der Substituenten Y¹, Y², Y³ und Z Halogen ist und die übrigen von ihnen Wasserstoff sind und W 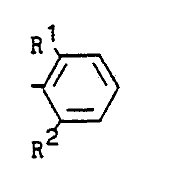 ist,
   dann entweder R¹ oder R² Alkyl oder Alkoxy ist,
(b) in dem Fall, in dem T -CONH-ist, Y¹, Y² und Y³ Wasserstoff sind, Z Halogenoalkoxy oder 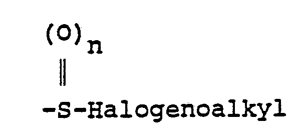 ist und W 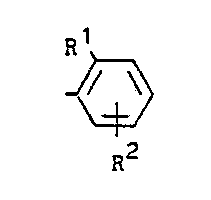 ist,
   dann entweder R¹ oder R² Alkoxy ist,
(c) in dem Fall, in dem T eine Einfachbindung ist. Y', Y² und Y³ Wasserstoff sind, Z Halogenoalkoxy oder 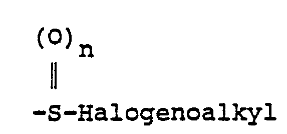 ist und W 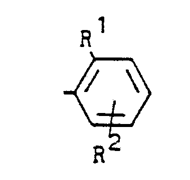 ist, dann R¹ und R² Alkoxy sind, oder
(d) in dem Fall, in dem T eine Einfachbindung ist, Y¹, Y² und Y³ Wasserstoff sind, Z Halogen ist und W 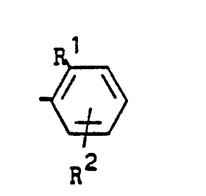 ist,
   dann entweder R' oder R² Alkoxy ist.

Die Verbindungen der Formel (I) werden mittels eines Verfahrens erhalten, bei dem
(a) in dem Fall, in dem X Sauerstoff ist und T eine Einfachbindung ist, Verbindungen der Formel (II) 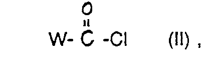 in der W die im Vorstehenden angegebenen Bedeutungen hat, mit Verbindungen der Formel 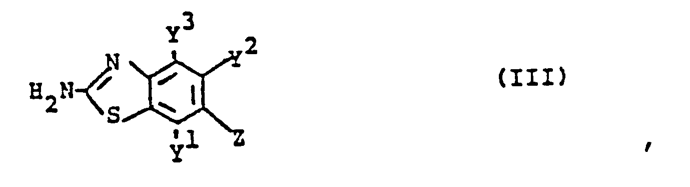 in der Y', Y², Y³ und Z die im Vorstehenden angegebenen Bedeutungen haben, in Gegenwart eines inerten Lösungsmittels und gegebenenfalls in Gegenwart einer Base umgesetzt werden, oder
b) in dem Fall. in dem X Schwefel ist und T eine Einfachbindung ist, Verbindungen der Formel (IV) 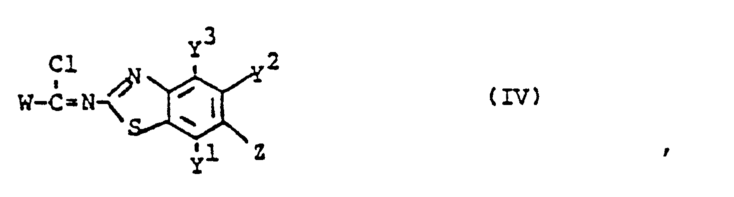 in der Y¹, Y², Y³, Z und W die im Vorstehenden angegebenen Bedeutungen haben, mit Hydrogensulfid in Gegenwart eines inerten Lösungsmittels und gegebenenfalls in Gegenwart einer Base umgesetzt werden, oder
c) in dem Fall. in dem T -CONH-ist. Verbindunnen der Formel (V) 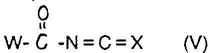 in der W und X die im Vorstehenden angegebenen Bedeutungen haben, mit den oben bezeichneten Verbindungen der Formel (III) in Gegenwart eines inerten Lösungsmittels umgesetzt werden.

Die neuen N-Benzothiazolylamide zeigen potente insektizide Eigenschaften.

Überraschenderweise zeigen die N-Benzothiazolylamide gemäß der Erfindung eine wesentlich größere insektizide Wirkung als Verbindungen, die aus dem oben angeführten Stand der Technik bekannt sind.

In den allgemeinen Formeln sind Alkyl oder die Alkyl-Teile der erwähnten Reste geradkettig oder verzweigt und enthalten vorzugsweise 1 bis 6, besonders bevorzugt 1 bis 4 und ganz besonders bevorzugt 1 bis 2 Kohlenstoff-Atome. Die Halogenoalkyl-Teile enthalten vorzugsweise 1 bis 5 und besonders bevorzugt 1 bis 4 Halogen-Atome, die gleich oder verschieden sein können (Halogen bedeutet Fluor, Chlor, Brom und lod, vorzugsweise Fluor oder Chlor). Als Beispiele erwähnt seien Methyl, Ethyl, n-und i-Propyl, n-, i-, sec-und tert-Butyl, Methoxy, Ethoxy, Methylthio, Methylsulfonyl, Trifluoromethyl, Trifluoromethoxy, Tetrafluoroethoxy und Trifluoromethylthio.

Aryl und die Aryl-Teile der betreffenden Reste enthalten vorzugsweise 6 oder 10 Kohienstoff-Atome. Besonders bevorzugt ist Phenyl. Die Alkyl-Teile in den Aralkyl-Komponenten enthalten vorzugsweise 1 bis 3, besonders bevorzugt 1 oder 2, Kohlenstoff-Atome. Als Beispiele erwähnt seien Phenyl, Phenyloxy, Phenylthio, Phenylsulfonyl, Benzyl, Phenylethyl und Benzylthio.

Alkenyl, Alkenyloxy, Alkinyloxy und Halogenoalkenyloxy enthalten vorzugsweise 2 bis 6 und besonders bevorzugt 2 bis 4 Kohlenstoff-Atome und als Halogen-Atome (vorzugsweise 1 bis 3) Fluor, Chlor und/oder Brom, vorzugsweise Fluor und/oder Chlor.

Die 5-bis 6-gliedrigen heterocyclischen Gruppen können gesättigt, ungesättigt oder heteroaromatisch sein. Sie enthalten 1 bis 3, vorzugsweise 1 oder 2, gleiche oder verschiedenartige Hetero-Atome. Als Hetero-Atome erwähnt seien Stickstoff, Sauerstoff und Schwefel. Die heterocyclischen Gruppen enthalten wenigstens ein Stickstoff-Atom. Als Beispiele erwähnt seien Pyridyl und Morpholinyl.

Halogen bezeichnet (sofern nichts anderes angegeben ist) Fluor, Chlor, Brom und lod, vorzugsweise Fluor, Chlor und Brom und insbesondere Fluor und Chlor.

Die im Vorstehenden als gegebenenfalls substituiert bezeichneten Reste können einen oder mehrere identische oder verschiedenartige Sobstituenten enthalten. Als bevorzugte Substituenten erwähnt seien Halogen wie Fluor, Chlor und Brom, vorzugsweise Chlor, C₁-C₄-Alkyl wie Methyl und Ethyl, Ci-C₄-Alkoxy wie Methoxy und Ethoxy, C₁-C₄-Alkylthio wie Methylthio und Ethylthio, C₁-C₄-Halogenoalkyl, Ci-C₄-Halogenoalkoxy und C₁-C₄-Halogenoalkylthio (die jeweils 1 bis 5 identische oder verschiedenartige Halogen-Atome, vorzugsweise Fluor, Chlor oder Brom, besonders bevorzugt Fluor oder Chlor enthalten), Cyano oder Nitro. Bevorzugte Substituenten sind Fluoro, Chloro und Bromo. C₁-C₄-Alkyl (insbesondere Methyl), C₁-C₄-Halogenoalkyl (insbesondere Trifluoromethyl, C₁-C₄-Halogenoalkoxy (insbesondere Trifluoromethoxy), C₁-C₄-Halo- genoalkylthio (insbesondere Trifluoromethylthio) und Cyano.
n bezeichnet 0, 1 oder 2, vorzugsweise 0.

Von den erfindungsgemäßen N-Benzothiazolylamiden der Formel (I) sind bevorzugte Verbindungen diejenigen, worin
X Sauerstoff oder Schwefel bezeichnet,
T eine Einfachbindung oder -CONH-bezeichnet,
Y¹ und Y² gleich oder verschieden sind und Wasserstoff, Fluoro, Chloro, Bromo oder C₁-C₄-Alkyl bezeichnen,
Y³ Wasserstoff bezeichnet,
Z C₁-C₄-Alkoxy, C₁-C₄-Halogenoalkoxy, C₂-C₄-Halogenoalkenyloxy, C₆-C₁₀-Aryl, Phenoxy, das durch wenigstens einen Substituenten ausgewählt aus Fluoro, Chloro, Bromo, Trifluoromethyl, Trifluoromethoxy, Trifluoromethylthio und Cyano substituiert sein kann, bezeichnet, oder
Z Phenylthio, das durch die oben für Phenoxy angegebenen Substituenten substituiert sein kann, 3-Chloro-5-trifluoromethyl-2-pyridyloxy oder eine Gruppe der Formel 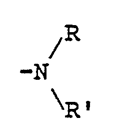 bezeichnet, worin R und R' identisch oder verschiedenartig sind und C₁-C₄-Alkyl oder C₂-C₃-Alkenyl bezeichnen und R und R' zusammen mit dem N-Atom, an das sie gebunden sind, eine gegebenenfalls substituierte 6-gliedrige heterocyclische Gruppe bilden können, die ein Sauerstoff-Atom neben einem Stickstoff-Atom enthalten kann, und
Z zusammen mit Y' oder Y² eine Tetrafluoroethylendioxy-Gruppe bilden kann und
W 2-Chlorophenyl, 2-Chloro-6-fluorophenyl oder 2,6-Difluorophenyl bezeichnet, mit der Maßgabe, daß wenigstens einer der Substituenten Y¹ und Y² Fluoro, Chloro, Bromo oder C₁-C₄-Alkyl bezeichnet.
Ganz besonders bevorzugte N-Benzothiazolylamide der Formel (I) sind diejenigen, worin
X Sauerstoff bezeichnet,
T eine Einfachbindung oder -CONH-bezeichnet,
Y¹ und Y² gleich oder verschieden sind und Wasserstoff oder Chloro bezeichnen,
Y³ Wasserstoff bezeichnet,
Z Ethoxy, 1,1,2,2-Tetrafluoroethoxy, Phenyl, Phenoxy, das durch wenigstens einen Substituenten ausgewählt aus Chloro, Trifluoromethyl und Trifluoromethoxy substituiert ist, bezeichnet, oder
Z Dimethylamino oder 2,6-Dimethylmorpholino ist und
W 2-Chlorophenyl oder 2,6-Difluorophenyl bezeichnet, mit der Maßgabe, daß wenigstens einer der Substituenten Y¹ und Y² Chloro bezeichnet.

Die folgenden Verbindungen seien speziell erwähnt:
N-[5,7-Dichloro-6-(1,1,2,2-tetrafluoroethoxy)benzothiazol-2-yl]-2,6-difluorobenzamid,
N-[5,7-Dichloro-6-(4-chlorophenoxy)benzothiazol-2-yl]-2,6-difluorobenzamid,
N-[5,7-Dichloro-6-(3-chloro-5-trifluoromethyl-2-pyridyloxy)benzothiazol-2-yl]-2,6-difluorobenzamid,
N-[6-(2-Chloro-4-trifluoromethylphenoxy)benzothiazol-2-yl]-2,6-difluorobenzamid,
N-(6,7-Tetrafluoroethylendioxybenzothiazol-2-yl)-2,6-difluorobenzamid,
N-(5,6-Tetrafluoroethylendioxybenzothiazol-2-yl)-2,6-difluorobenzamid,
N-[5,7-Dichloro-6-(1,1,2,2-tetrafluoroethoxy)benzothiazol-2-yl]-2-chlorobenzamid,
N-[5,7-Dichloro-6-(2,4-dichlorophenoxy)benzothiazol-2-yl]-2-chlorobenzamid,
N-[5,7-Dichloro-6-(3-chloro-5-trifluoromethyl-2-pyridyloxy)benzothiazol-2-yl]-2-chlorobenzamid,
N-(6,7-Tetrafluoroethylendioxybenzothiazol-2-yl)-2-chlorobenzamid,
N-(5,6-Tetrafluoroethylendioxybenzothiazol-2-yl)-2-chlorobenzamid,
N-[5,7-Dichloro-6-(4-trifluoromethylphenoxy)benzothiazol-2-yl]-2,6-difluorobenzamid,
N-[5.7-Dichloro-6-(4-trifluoromethoxyphenoxy)benzothiazol-2-yl]-2.6-difluorobenzamid,
N-[6-(2-Chloro-4-trifluoromethoxyphenoxy)benzothiazol-2-yl]-2,6-difluorobenzamid,
N-[6-(2-Chloro-4-trifluoromethylphenoxy)benzothiazol-2-yl]-2,6-chlorobenzamid,
N-[6-(2-Chloro-4-trifluoromethoxyphenoxy)benzothiazol-2-yl]-2,6-chlorobenzamid,
1-(2,6-Difluorobenzoyl)-3-[6-(2-chloro-4-trifluoromethylphenoxy)benzothiazol-2-yl]harnstoff,
1-(2.6-Difluorobenzoyl)-3-[6-(2-chloro-4-trifluoromethoxyphenoxy)benzothiazol-2-yl]harnstoff,
1-(2-Chlorobenzoyl)-3-[6-(2-chloro-4-trifluoromethylphenoxy)benzothiazol-2-yl]harnstoff und
1-(2-Chlorobenzoyl)-3-[6-(2-chloro-4-trifluoromethoxyphenoxy)benzothiazol-2-yl]harnstoff.

Wenn 2,6-Difluorobenzoylchlorid und 2-Amino-5,7-dichloro-6-)1,1,2,2-tetrafluoroethoxy)benzothiazol als Ausgangsstoffe in dem Verfahren a) eingesetzt werden, wird die Reaktion durch das nachstehende Schema veranschaulicht.

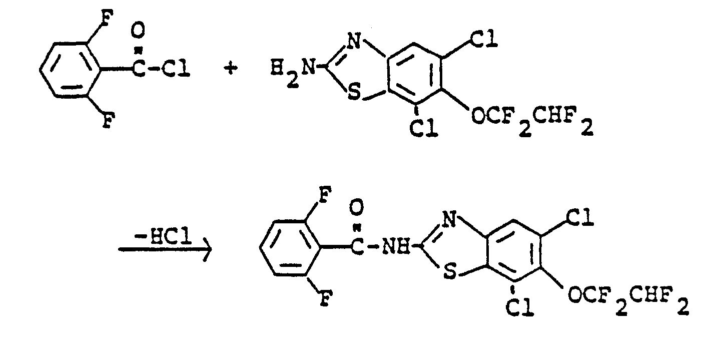

Wenn N-[5,7-Dichloro-6-(4-trifluoromethylthiophenoxy)benzothiazol-2-yl]-2-chlorobenzimidoylchlorid und Hydrogensulfid als Ausgangsstoffe in dem Verfahren b) eingesetzt werden, wird die Reaktion schematisch wie folgt dargestellt.

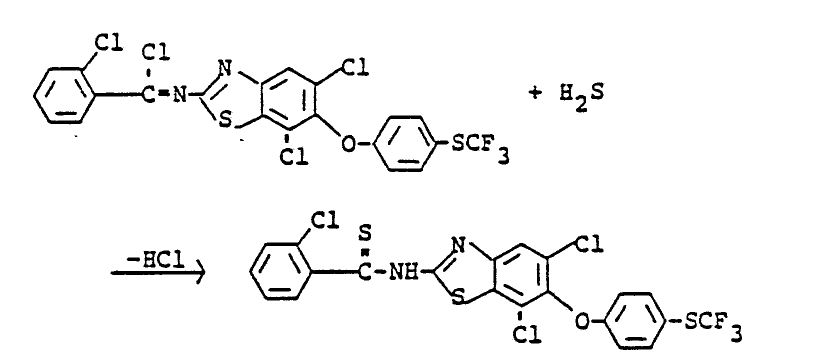

Wenn 2-Chlorobenzoylisocyanat und 2-Amino-6-phenoxybenzothiazol als Ausgangsstoffe in dem Verfahren c) eingesetzt werden, wird die Reaktion durch das nachstehende Schema veranschaulicht.

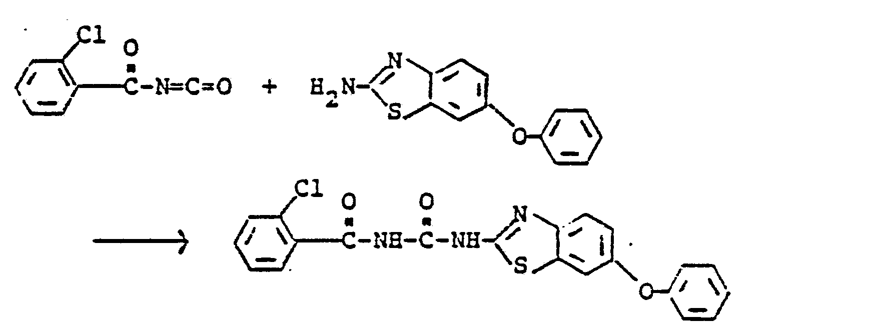

In dem Verfahren a) basieren die Verbindungen der Formel (II) als Ausgangsstoffe auf den oben angegebenen Definitionen für W.

In der Formel (11) ist W vorzugsweise gleichbedeutend mit den vorstehenden bevorzugten Definitionen.

Die Verbindungen der Formel (II) sind wohlbekannt, und zu speziellen Beispielen zählen 2-Chlorobenzoylchlorid,
2-Chloro-6-fluorobenzoylchlorid,
2,6-Difluorobenzoylchlorid,
2-Chloronicotinylchlorid,
2-Methylbenzoylchlorid,
2-Chloro-4-fluorobenzoylchlorid und
2,6-Dimethoxybenzoylchlorid.

Die Verbindung der Formel (III) bedeuten solche, die auf den vorstehenden Definitionen für Y', Y², Y³ und Z beruhen.

In der Formel (III) sind Y', Y², Y³ und Z vorzugsweise gleichbedeutend mit den vorstehenden bevorzugten Definitionen.

Die Formel (III) schließt neue Verbindungen ein, die speziell als Verbindungen der nachstehenden Formel (III') 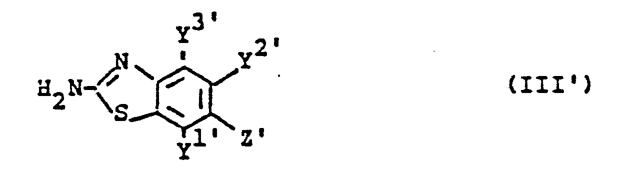 vorgeschlagen werden, worin
Y^{1'} und Y^{2'} gleich oder verschieden sind und Wasserstoff, Halogen oder Alkyl bezeichnen,
Y 3' Wasserstoff ist und in dem Fall, in dem Y^{1'} und Y^{2'} Halogen oder Alkyl sind, dann
Z' Alkoxy, Halogenoalkoxy, Aryloxy, Arylthio, gegebenenfalls substituiertes Pyridyloxy oder eine Gruppe der Formel 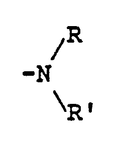 ist, worin R und R' jeweils Alkyl oder Alkenyl bezeichnen und R und R_{'} zusammen mit dem N-Atom, an das sie gebunden sind, eine gegebenenfalls substituierte 5-bis 6-gliedrige heterocyclische Gruppe bilden können, die Hetero-Atome neben einem Stickstoff-Atom enthalten kann, oder in dem Fall, in dem entweder Y^{1'} oder Y^{2'} Wasserstoff ist dann
Z' 2-Chioro-4-trifluoromethylphenoxy, 2-Chloro-4-trifluoromethoxyphenoxy, 4-Trifluoromethylphenoxy oder 4-Trifluoromethoxyphenoxy ist und
Z' zusammen mit Y^{1'} oder Y^{2'} eine Tetrafluroethylendioxy-Gruppe bilden kann.

Die bevorzugten Definitionen für Y^{1'}, Y ^{2'}, Y³ und Z' entsprechen den angegebenen bevorzugten Definitionen für Y', Y², Y³ und Z für die Verbindungen der Formel (I).

Die Verbindungen der Formel (III), einschließlich derjenigen der Formel (III') können leicht erhalten werden, beispielsweise mit Hilfe eines Verfahrens, bei dem
d) Verbindungen der Formel (VI) 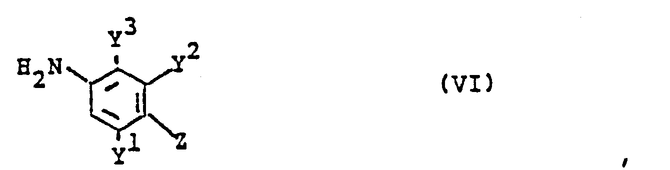
   in der Y¹, Y², Y³ und Z die oben angegebenen Bedeutungen haben, mit einem Thiocyanat-Salz, etwa Kaliumthiocyanat, und Brom umgesetzt werden oder
e) Verbindungen der Formel (VII) 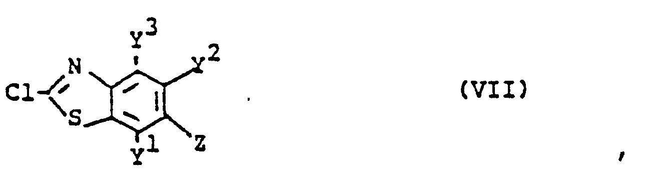
   in der Y', Y², Y³ und Z die oben angegebenen Bedeutungen haben, mit Ammoniak umgesetzt werden oder
f) Verbindungen der Formel (VIII) 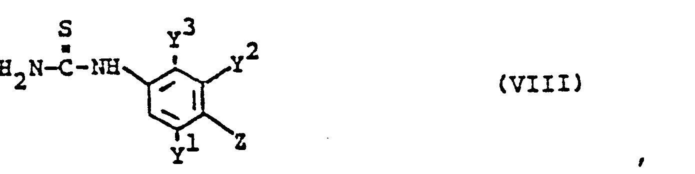
   in der Y¹, Y², Y³ und Z die oben angegebenen Bedeutungen haben, reduzierend cyclisiert werden oder
g) Verbindungen der Formel (IX) 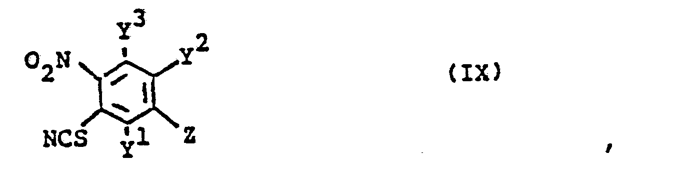
   in der Y¹, Y², Y³ und Z die oben angegebenen Bedeutungen haben, reduzierend cyclisiert werden.

Das Verfahren d) kann entsprechend der Offenbarung der FR-PS 1 502 178 durchgeführt werden.

Das Verfahren e) kann nach einer in einer bekannten Veröffentlichung, Yakugaku Zasshi, Band 78, Seiten 437-438, beschriebenen Methode durchgeführt werden. Die Verbindungen der Formel (VII) umfassen bekannte Verbindungen, die in der JP-OS 135 481/1981 und in der EP-OS 0 043 573 beschrieben sind.

Das Verfahren f) kann in einfacher Weise nach der in der JP-OS 59 679/1984 oder in Org. Synth., Band 22, Seiten 16-19, beschriebenen Methode durchgeführt werden.

Das Verfahren g) kann in einfacher Weise durchgeführt werden, beispielsweise nach der in J. Chem. Soc. 1969, Seiten 268-272, beschriebenen Methode.

Wenn in der Formel (III) Y' und Y² gleich sind und Z von Wasserstoff verschieden ist oder Y³ und Z von Wasserstoff verschieden sind, kann die Verbindung der Formel (III) leicht in guten Ausbeuten nach dem Verfahren d) erhalten werden.

Zu speziellen Beispielen für die Verbindung der Formel (III) zählen
2-Amino-5,7-dichloro-6-(1,1,2,2-tetrafluoroethoxy)benzothiazol,
2-Amino-5,7-dichloro-6-(4-chlorophenoxy)benzothiazol,
2-Amino-5,7-dichloro-6-(3-chloro-5-trifluoromethyl-2-pyridyloxy)benzothiazol,
2-Amino-6-(2-chloro-4-trifluoromethylphenoxy)benzothiazol,
2-Amino-6,7-tetrafluoroethylendioxybenzothiazol,
2-Amino-5,6-tetrafluoroethylendioxybenzothiazol,
2-Amino-6-(3.chloro-5-trifluoromethyl-2-pyridyloxy)benzothiazol,
2-Amino-6-)2-chloro-4-trifluoromethoyphenoxy)benzothiazol und
2-Amino-5,7-dichloro-6-(2,4-dichlorophenoxy)benzothiazol.

In dem Verfahren b) sind die Ausgangs-Verbindungen solche, die auf den vorstehenden Definitionen für Y¹, Y², Y³, Z und W beruhen.

Die Verbindungen der Formel (IV) können in einfacher Weise dadurch erhalten werden, daß die Verbindungen der Formel (la) gemäß vorliegenden Erfindung, die mit Hilfe des Verfahrens a) synthetisiert wurden, mit Phosphorpentachlorid umgesetzt werden.

Bei der praktischen Durchführung des Verfahrens zur Herstellung der Verbindung der Formel (IV) wird 1 mol der Verbindungen der Formel (la) mit, beispielsweise, 1 bis etwa 1,2 mol Phosphorpentachlorid bei einer Temperatur von etwa 80 °C bis etwa 130 °C während etwa 0,1 bis 5 h in Gegenwart eines inerten Lösungsmittels, zum Beispiel Benzol, Toluol, Xylol oder eines gesättigten Kohlenwasserstoffs wie Hexane, eines chlorierten Kohlenwasserstoffs wie Chloroform oder eines Ethers wie Dioxan umgesetzt, wodurch die Verbindungen der Formel (IV) erhalten werden.

Die Verbindungen der Formel (V) sind wohlbekannt und beispielsweise in J. Agr. Food Chem., Band 21, Seiten 348-354, beschrieben. Spezielle Beispiele sind
2-Chlorobenzoylisocyanat,
2,6-Difluorobenzoylisocyanat,
2-Chloro-6-fluorobenzoylisocyanat,
2-Chloro-4-fluorobenzoylisocyanat,
2-Methylbenzoylisocyanat und
2-Chloro-3-pyridylcarbonylisocyanat.

In der Praxis des Verfahrens a) können alle inerten Lösungsmittel als geeignete Verdünnungsmittel verwendet werden.

Zu Beispielen der Verdünnungsmittel zählen Wasser; aliphatische, alicyclische oder aromatische (gegebenenfalls chlorierte) Kohlenwasserstoffe wie Hexan, Cyclohexan, Petrolether, Ligroin, Benzol, Toluol, Xylol, Methylenchlorid, Chloroform, Kohlenstofftetrachlorid, Ethylenchlorid, Trichloroethylen und Chlorbenzol; Ether wie Diethylether, Methylethylether, Diisopropylether, Dibutylether, Propylenoxid, Dioxan und Tetrahydrofuran; Ketone wie Aceton, Methylethylketon, Methylisopropylketon und Methylisobutylketon; Nitrile wie Acetonitril, Propionitril und Acrylnitril; Ester wie Ethylacetat und Amylacetat; Säureamide wie Dimethylformamid, N-Methylpyrrolidon und Dimethylacetamid; Sulfone und Sulfoxide wie Dimpthylsulfoxid und Sulfolan; und Basen wie Pyridin.

Das obige Verfahren a) kann in Gegenwart einer Base durchgeführt werden. Beispiele für die Base sind organische tertiäre Amine wie Triethylamin, Dimethylanilin, Pyridin und Lutidin und anorganische Basen, beispielsweise Alkalimetallhydroxide und -carbonate wie Natriumhydroxid, Kaliumhydroxid, Natriumcarbonat und Kaliumcarbonat.

Das obige Verfahren kann in einem weiten Temperaturbereich durchgeführt werden, beispielsweise bei einer Temperatur zwischen etwa -20 °C und dem Siedepunkt der Mischung, vorzugsweise zwischen etwa 0 °C und etwa 100 °C. Die Reaktion wird vorzugsweise unter Atmosphärendruck durchgeführt, jedoch ist es auch möglich, bei erhöhtem oder vermindertem Druck zu arbeiten.

Bei der praktischen Durchführung des Verfahrens a) können die gewünschten neuen Verbindungen der Formel (I) beispielsweise dadurch erhalten werden, daß 1 mol der Verbindungen der Formel (II) mit etwa 0,8 bis 1,2 mol, vorzugsweise etwa 0,9 bis 1 mol, der Verbindungen der Formel (III) in einem inerten Lösungsmittel in Gegenwart einer Base umgesetzt werden.

Wenn in dem Verfahren a) eine Base nicht verwendet wird, können die gewünschten Verbindungen der Formel (I) dadurch erhalten werden, daß die Verbindungen der Formel (11) und die Verbindungen der Formel (111) in einem Stoffmengenverhältnis ("Molverhältnis") 1 : 1 umgesetzt werden oder eine von ihnen in einem molaren Überschuß mit einer Zunahme oder Abnahme innerhalb von 5 % bei einer Temperatur von wenigstens 120 °C in Gegenwart eines inerten Lösungsmittels, beispielsweise eines gegebenenfalls chlorierten aromatischen oder aliphatischen Kohlenwasserstoffs mit einem Siedepunkt von wenigstens 120 °C wie Xylol, Chlorobenzol oder Dichlorobenzol, eingesetzt wird.

Die gleichen inerten Lösungsmittel, die oben im Hinblick auf das Verfahren a) angegeben sind, können bei der Durchführung des Verfahrens b) verwendet werden.

Das Verfahren b) kann in Gegenwart einer Base durchgeführt werden.

Als Base können allgemein anorganische und organische Basen eingesetzt werden, die nicht mit den Ausgangs-Imidoylchloriden der Formel (IV) reagieren oder Schwierigkeiten haben, mit diesen unter speziellen Bedingungen zu reagieren, beispielsweise Hydroxide, Carbonate, Hydrogencarbonate und Alkoholate von Alkalimetallen und tertiäre Amine wie Triethylamin, Dimethylanilin, Pyridin und Lutidin.

Das Verfahren b) kann in einem weiten Temperaturbereich durchgeführt werden, beispielsweise bei einer Temperatur zwischen etwa -20 °C und etwa 100 °C, vorzugsweise zwischen etwa 0 °C und etwa 80 °C. Zweckmäßigerweise wird die Reaktion unter Atmosphärendruck durchgeführt, jedoch ist es auch möglich, bei erhöhtem oder vermindertem Druck zu arbeiten.

Bei der praktischen Durchführung des Verfahrens b) können die gewünschten Verbindungen der Formel (I) dadurch erhalten werden, daß 1 mol der Verbindungen der Formel (IV) mit 1 bis etwa 1.5 mol, vorzugsweise 1 bis etwa 1,2 mol, Hydrogensulfid in Gegenwart eines inerten Lösungsmittels und einer Base umgesetzt werden.

Die mittels des Verfahrens b) synthetisierten Verbindungen der Formel (I) können sowohl die Enol-Form als auch die Keto-Form wie folgt annehmen:

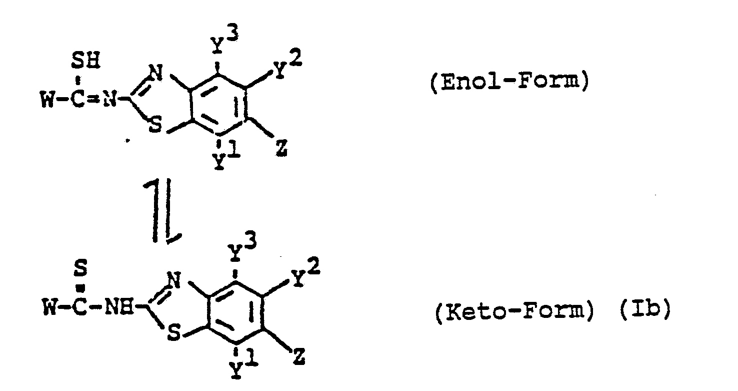

In den Formeln haben Y¹, Y², Y³, Z und W die oben angegebenen Bedeutungen.

In der Praxis des Verfahrens c) können alle oben beispielhaft im Hinblick auf das Verfahren a) genannten inerten Lösungsmittel mit Ausnahme von Wasser eingesetzt werden.

Das Verfahren c) kann in einem weiten Temperaturbereich durchgeführt werden, beispielsweise bei einer Temperatur zwischen etwa 0 °C und etwa 100 °C, vorzugsweise zwischen etwa 10 °C und etwa 40 °C. Die Reaktion wird vorzugsweise unter Atmosphärendruck durchgeführt, jedoch ist es auch möglich, bei erhöhtem oder vermindertem Druck zu arbeiten.

Bei der praktischen Durchführung des Verfahrens c) können die gewünschten neuen Verbindungen der Formel (I) dadurch erhalten werden, daß 1 mol der Verbindungen der Formel (V) mit etwa 0,8 bis 1,2 mol. vorzugsweise etwa 0,9 bis 1 mol, der Verbindungen der Formel (111) in einem inerten Lösungsmittel umgesetzt werden.

Die aktiven Verbindungen werden von Pflanzen gut vertragen, haben einen günstigen Wert der Toxizität gegenüber warmblütigen Tieren und lassen sich einsetzen zur Bekämpfung von Arthropoden-Schädlingen, insbesondere von Insekten, die in der Land-und Forstwirtschaft auftreten, zum Schutz von Lagerprodukten und Materialien und auf dem Gebiet der Hygiene. Sie sind gegenüber normal empfindlichen und resistenten Species sowie gegen alle oder einige Entwicklungsstadien aktiv. Zu den oben genannten Schädlingen zählen:
Aus der Klasse der Isopoda beispielsweise Oniscus asellus, Armadillidium vulgare und Porcellio scaber;
aus der Klasse der Diplopoda beispielsweise Blaniulus guttulatus;
aus der Klasse der Chilopoda beispielsweise Geophilus carpophagus und Scutigera spec.;
aus der Klasse der Symphyla beispielsweise Scutigerella immaculata;
aus der Ordnung der Thysanura beispielsweise Lepisma sacharina;
aus der Ordnung der Collembola beispielsweise Onychiurus armatus;
aus der Ordnung der Orthoptera beispielsweise Blatta orientalis, Periplaneta americana, Leucophaea maderae, Blattella germanica, Acheta domesticus, Gryllotalpa spp., Locusta migratoria migratorioides, Melanoplus differentialis und Schistocerca gregaria;
aus der Ordnung der Dermaptera beispielsweise Forficula auricularia,
aus der Ordnung der Isoptera beispielsweise Reticulitermes spp.,
aud der Ordnung der Anoplura beispielsweise Phylloxera vastatrix, Pemphigus spp., Pediculus humanus corporis, Haematopinus spp. und Linognathus spp.;
aus der Ordnung der Mallophaga beispielsweise Trichodectes spp. und Damalinea spp,;
aus der Ordnung der Thysanoptera beispielsweise Hercinothrips femoralis und Thrips tabaci;
aus der Ordnung der Heteroptera beispielsweise Eurygaster spp., Dysdercus intermedius, Piesma quadrata, Cimex lectularius, Rhodnius prolixus und Triatoma spp.;
aus der Ordnung der Homoptera beispielsweise Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humili, Rhopalosiphum padi. Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. und Psylla spp.,
aus der Ordnung der Lepidoptera beispielsweise Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp.. Spodoptera exigua, Mamestra brassicae, Panolis flammea, Prodenia litura, Spodoptera spp., Trichoplusia ni. Carpocapsa pomonella, Pieris spp., Chilo spp., Pyrausta nubilalis, Ephestia kuehniella, Galleria melloneila, Cacoecia podana, Capua reticulana, Choristoneura fumiferana, Clysia ambiguella, Homona magnanima und Tortrix viridana;
aus der Ordnung der Coleoptera beispielsweise Anobium punctatum, Rhizopertha dominica, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis und Costelytra zealandica;
aus der Ordnung der Hymenoptera beispielsweise Diprion spp., Hoplocampa spp., Lasius spp., Monomorium pharaonis und Vespa spp.;
aus der Ordnung der Diptera beispielsweise Aedes spp., Anopheles spp., Culex spp., Drosophila melanogaster, Musca spp.. Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae und Tipula paludosa;
aus der Ordnung der Siphonaptera beispielsweise Xenopsylla cheopis und Ceratophyllus spp.; aus der Klasse der Arachnida beispielsweise Scorpio maurus und Latrodectus mactans;
aus der Ordnung der Aranina beispielsweise Acarus siro, Argas spp.. Ornithodoros spp., Dermanyssus gallinae; Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp. und Tetranychus spp..

Auf dem Gebiet der Viehzucht und -aufzucht sind die neuen Verbindungen der Formel (I) gegen verschiedene schädliche Tierparasiten (innere und äußere Parasiten) wie Insekten und Würmer wirksam.
Beispielen für solche Tierparasiten sind Insekten wie
Gastrophilus spp.,
Stomoxys spp.,
Trichodectes spp.
Rhodnius spp. und
Ctenocephalides canis.

Die aktiven Verbindungen können in gebräuchliche Formulierungen überführt werden. etwa Lösungen, Emulsionen, Suspensionen, Pulver, Schäume, Pasten, Granulat, Aerosol, mit der aktiven Verbindung imprägnierte natürliche oder synthetische Stoffe, sehr feine Kapseln in polymeren Substanzen, Überzugsmassen zur Verwendung auf Saatgut (Beizmittel) sowie Formulierungen für den Einsatz mit Verbrennungseinrichtungen wie Räucherpatronen, Räucher dosen und Räucherschlangen sowie für die kalte Vernebelung und die warme Vernebelung nach dem Ultra-Low-Volume-Verfahren.

Diese Formulierungen können in bekannter Weise hergestellt werden, beispielsweise durch Vermischen der aktiven Verbindungen mit Streckmitteln, das heißt mit flüssigen oder verflüssigten gasförmigen oder festen Verdünnungsmitteln oder Trägern, gegebenenfalls unter Verwendung grenzflächenaktiver Mittel, das heißt von Emulgatoren und/oder Dispergiermittein und/oder schaumbildenden Mitteln. Bei Verwendung von Wasser als Streckmittel können organische Lösungsmittel beispielsweise als Hilfslösungsmittel verwendet werden.

Als flüssige Lösungsmittel, Verdünnungsmittel oder Träger vorzugsweise geeignet sind aromatische Kohlenwasserstoffe wie Xylol, Toluol oder Alkylnaphthaline, chlorierte aromatische oder chlorierte aliphatische Kohlenwasserstoffe wie Chlorbenzole, Chloroethylene oder Methylenchlorid, aliphatische Kohlenwasserstoffe wie Cyclohexan oder Paraffine, beispielsweise Mineralöl-Fraktionen. Alkohole wie Butanol oder Glycol sowie deren Ether und Ester, Ketone wie Aceton, Methylethylketon, Methylisobutylketon oder Cyclohexanon oder stark polare Lösungsmittel wie Dimethylformamid und Dimethylsulfoxid sowie auch Wasser.

Unter verflüssigten gasförmigen Verdünnungsmitteln oder Trägern sind Flüssigkeiten zu verstehen, die bei normaler Temperatur und normalem Druck gasförmig wären, beispielsweise Aerosol-Treibmittel wie halogenierte Kohlenwasserstoffe sowie Butan, Propan, Stickstoff und Kohlenstoffdioxid.

Als feste Träger verwendbar sind gemahlene natürliche Minerale wie Kaoline, Tone, Talkum, Kreide, Quarz, Attapulgit, Montmorillonit oder Diatomeenerde und gemahlene synthetische Minerale wie hochdisperse Kieselsäure, Aluminiumoxid und Silicate. Als feste Träger für Granulat können zerkleinerte und fraktionierte Natursteinmaterialien verwendet werden, etwa Calcit, Marmor, Bimsstein, Sepiolith und Dolomit sowie synthetisches Granulat aus anorganischen und organischen Mehlen und Granulat aus organischem Material wie Sägemehl, Kokosnußschalen, Maiskolben und Tabakstengel.

Als emulgierende und/oder schaumbildende Mittel können nicht-ionische und anionische Emulgatoren wie Polyoxyethylenfettsäureester, Polyoxyethylenfettalkoholether, beispielsweise Alkylarylpolyglycol-Ether, Alkysulfonate, Alkylsulfate, Arylsulfonate sowie Albumin-Hydrolyseprodukte verwendet werden. Zu Dispergiermitteln zählen beispielsweise Ligninsulfit-Ablaugen und Methylcellulose.

Haftmittel wie Carboxymethylcellulose und natürliche und synthetische Polymere in Form von Pulvern, Granulat oder Latices wie Gummi arabicum, Polyvinylalkhol und Polyvinylacetat können bei der Formulierung verwendet werden.

Es ist möglich, farbgebehde Mittel, etwa anorganische Pigmente wie beispielsweise Eisenoxid, Titanoxid und Preußisch Blau und organische Farbstoffe wie Alizarin-Farbstoffe, Azo-Farbstoffe oder Metallphthalocyanin-Farbstoffe, sowie Spuren-Nährstoffe wie Salze von Eisen, Mangan, Bor, Kupfer, Cobalt, Molybdän und Zink zu verwenden.

Die Formulierungen enthalten im allgemeinen 0,1 bis 95 Gew.-%, vorzugsweise 0,5 bis 90 Gew.-% der aktiven Verbindung.

Die aktiven Verbindungen gemäß der Erfindung können in ihren handelsüblichen Formulierungen oder den aus diesen Formulierungen hergestellten Anwendungsformen im Gemisch mit anderen aktiven Verbindungen vorliegen, etwa mit Insektiziden, Ködern, Sterilisationsmitteln, Akariziden, Nematiziden, Fungiziden, Wachstumsregulatoren oder Herbiziden. Zu den Insektiziden gehören beispielsweise Phosphate, Carbamate, Carboxylate, chlorierte Kohlenwasserstoffe, Phenylharnstoffe und von Mikroorganismen erzeugte Substanzen. ⁻

Die aktiven Verbindungen gemäß der Erfindung können weiterhin in ihren handelsüblichen Formulierungen oder den aus diesen Formulierungen hergestellten Anwendungsformen in Gemisch mit synergistischen Mitteln vorliegen. Synergistische Mittel sind Verbindungen, die die Wirkung der aktiven Verbindungen steigern, ohne daß es für das zugesetzte synergistische Mittel erforderlich ist, selbst aktiv zu sein.

Der Gehalt der aktiven Verbindung in den aus den im Handel erhältlichen Formulierungen hergestellten Anwendungsformen kann innerhalb weiter Grenzen variieren. Die Konzentration der aktiven Verbindung in den Anwendungsformen kann, 0,0000001 bis 95 Gew -°ö betragen und liegt vorzugsweise zwischen 0,0001 und 1 Gew.-%.

Die Verbindungen werden in üblicher Weise in einer den Anwendungsformen angemessenen Form zur Anwendung gebracht.

Bei der Verwendung gegen Hygiene-Schädlinge und Schädlinge in Produkt-Vorräten zeichnen sich die aktiven Verbindungen durch eine hervorragende Rückstandswirkung auf Holz und Ton sowie eine gute Beständigkeit gegen Alkali auf gekalkten Unterlagen aus.

Die folgenden Beispiele erläutern die vorliegende Erfindung im einzelnen. Die vorliegende Erfindung ist jedoch nicht allein auf diese Beispiele beschränkt.

### Herstellungsbeispiele

### Beispiel 1

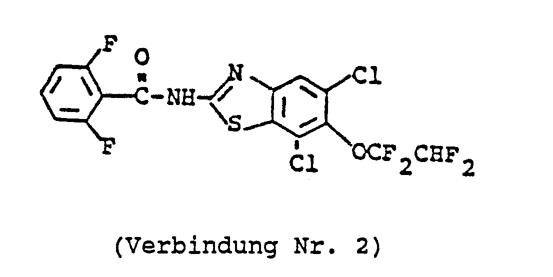

Eine Mischung aus 2-Amino-5,7-dichloro-6-(1,1,2,2-tetrafluoroethoxy)benzothiazol (3,35 g), Chlorobenzol (20 ml)und 2,6-Difluorobenzoylchlorid (1,8 g) wurde 3 h zum Rückfluß erhitzt. Im Anfangsstadium des Erhitzens zum Rückfluß wurde kräftig Hydrogenchlorid entwickelt. Die Entwicklung hörte jedoch bald auf, und die Reaktionsmischung wurde klar. Die Reaktionsmischung wurde über Nacht bei Raumtemperatur stehen gelassen. Die entstandenen Kristalle wurden durch Filtration gesammelt und mit Toluol (20 ml) gewaschen, wonach das gewünschte N-[5,7-Dichforo-6-(1,1,2.2-tetrafluoroethoxy)benzothiazol-2-yl]-2,6-di- fluorobenzamid (4,2 g) erhalten wurde; Schmp. 231-232 °C.

### Beispiel 2

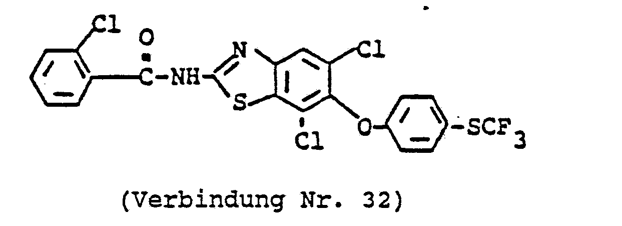

2-Amino-5,7-dichloro-6-(4-trifluoromethylthiophenoxy)benzothiazol (4,11 g) wurde in Tetrahydrofuran (80 ml) gelöst, und Triethylamin (1,1 g) wurde der Lösung zugesetzt. Bei 5 °C bis 10 °C wurde 2-Chlorobenzoylchlorid (1,75 g) hinzugefügt, und die Mischung wurde 5 h bei 20 °C bis 30 °C gerührt. Das Tetrahydrofuran wurde unter vermindertem Druck abgedampft. Der Rückstand wurde mit Wasser gewaschen und aus Ethanol umkristallisiert, wonach N-[5,7-Dichioro-6-)4-trifluoromethylthiophenoxy)benzothiazol-2-yl]-2-chlorobenzamid (4,1 g) erhalten wurde: Schmp. 211-213 °C.

### Beispiel 3

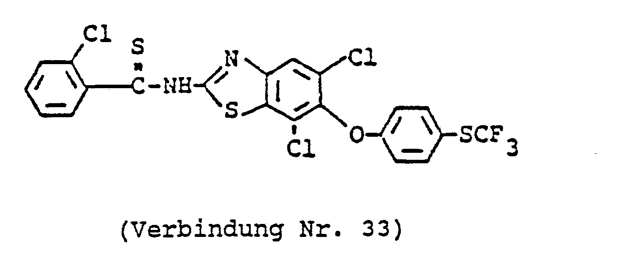

Eine Mischung aus der in Beispiel 2 erhaltenen Verbindung (2,2 g), Phosphorpentachlorid (0,92 g) und Toluol (60 ml) wurde 10 min zum Rückfluß erhitzt, und danach wurde etwa 20 min Hydrogensulfid in die Reaktionsmischung eingeleitet, bis die Erzeugung von Hydrogen chlorid aufhörte. Die Reaktionsmischung wurde bei 50 °C bis 60 °C filtriert. Das Filtrat wurde konzentriert, und der Rückstand wurde aus einer Mischung von Toluol und Hexan umkristallisiert, wordurch Kristalle (1,8 g) erhalten wurden. Da diese Kristalle eine Mischung aus der gewünschten Verbindung und der Oxo-Verbindung waren, wurde die Mischung (0.9 g) durch Säulenchromatographie unter Verwendung von Silicagel als Träger und Tetrahydrofuran_{/}Triethylamin (300;20, Vol./Nol.) als entwickelndes Lösungsmittel gereinigt, wonach N-[5,7-Dichloro-6-{4-trifluoromethylthiophenoxy)benzothiazol-2-yl]-2-chlorobenzothioamid (0,3 g) als gewünschte Verbindung erhalten wurde; Schmp. 272-273 °C.

### Beispiel 4

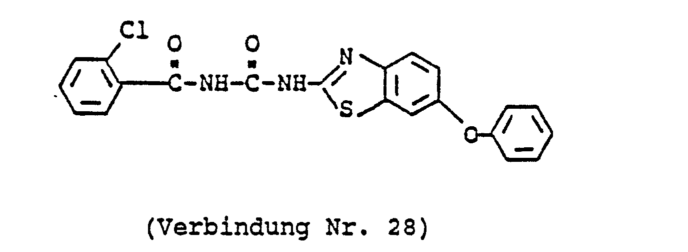

2-Amino-6-phenoxybenzothiazol (1 g) wurde in Tetrahydrofuran (50 ml) gelöst, und bei weniger als 10 °C wurde eine Lösung von 2-Chlorobenzoylisocyanat (0,75 g) in Toluol (10 ml) tropfenweise hinzugefügt. Die Mischung wurde bei Raumtemperatur 4 h gerührt, und dann wurde das Tetrahydrofuran unter vermindertem Druck abdestilliert. Der Rückstand wurde mit Toluol vermischt, und die resultierenden Kristalle wurden durch Filtration gesammelt. wonach 1-(2-Chlorobenzoyl)-3-(6-phenoxybenzothiazol-2-yl)-harnstoff (1,5 g) erhalten wurde: Schmp. 286-290 °C.

Die nachstehende Tabelle 1 zeigt Verbindungen der Formel (I) gemäß der vorliegenden Erfindung, die nach den gleichen Methoden wie in den vorstehenden Beispielen 1, 2, 3 und 4 erhalten wurden, zusammen mit den in den Beispielen 1 bis 4 erhaltenen Verbindungen.

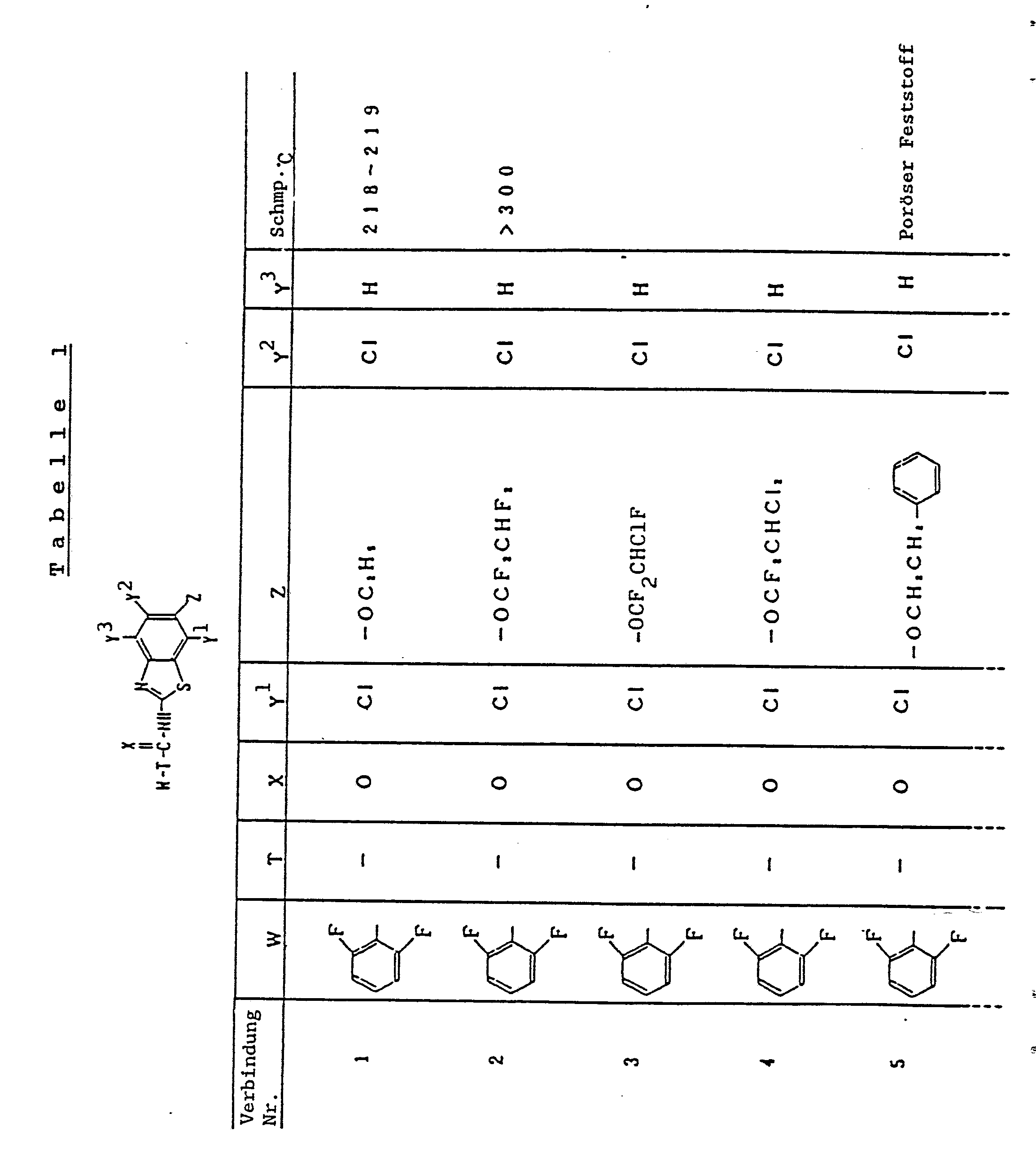

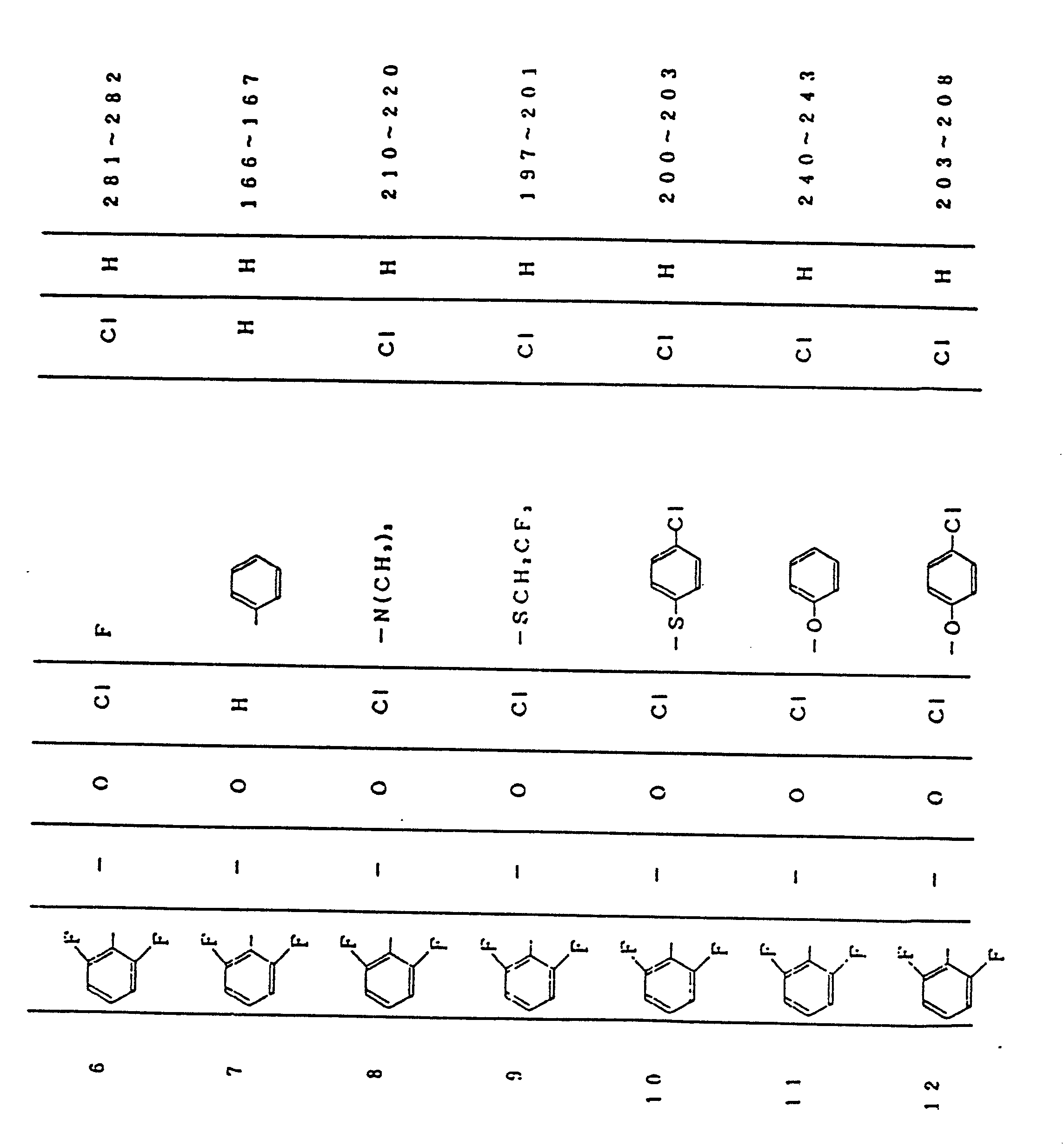

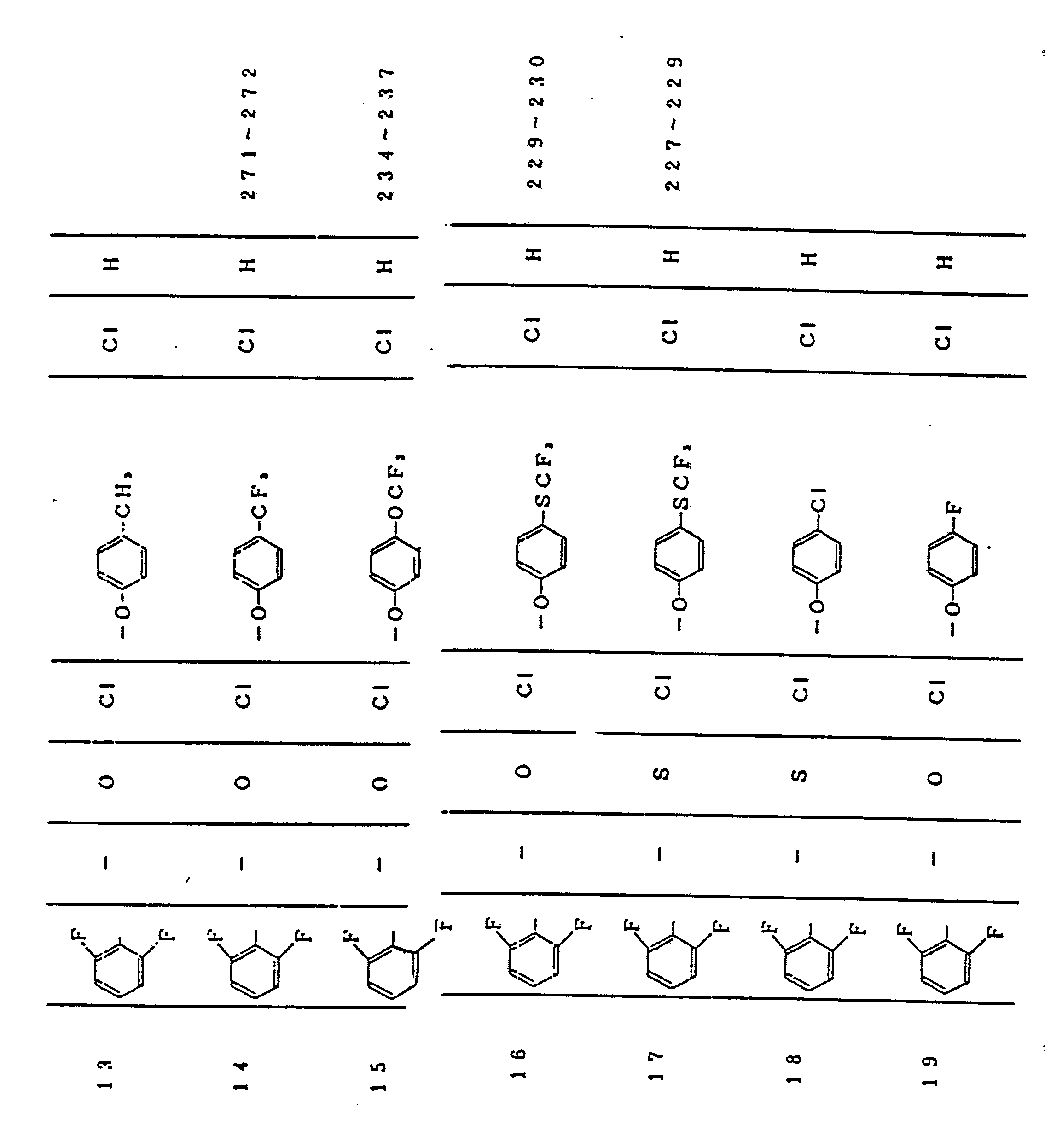

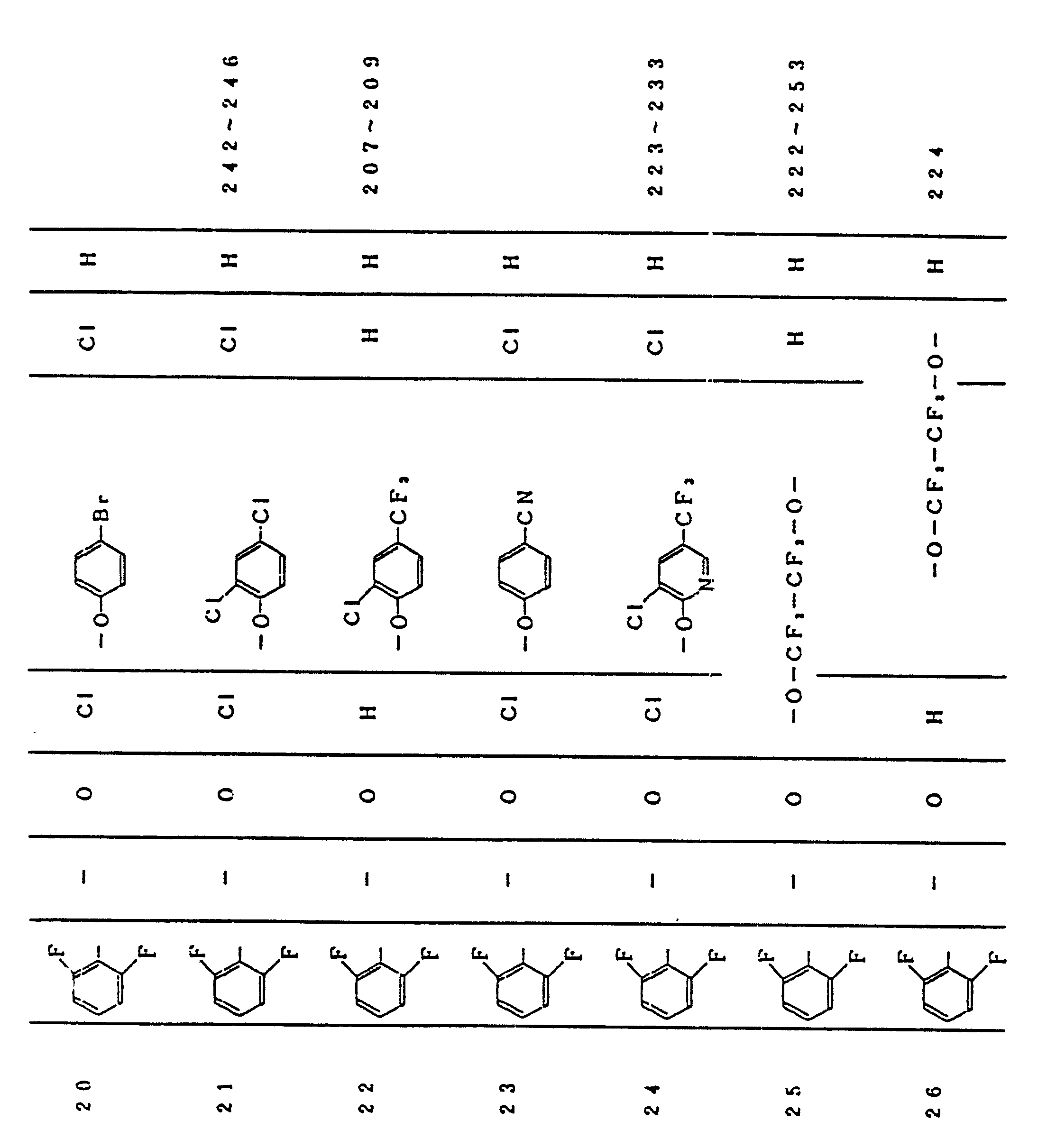

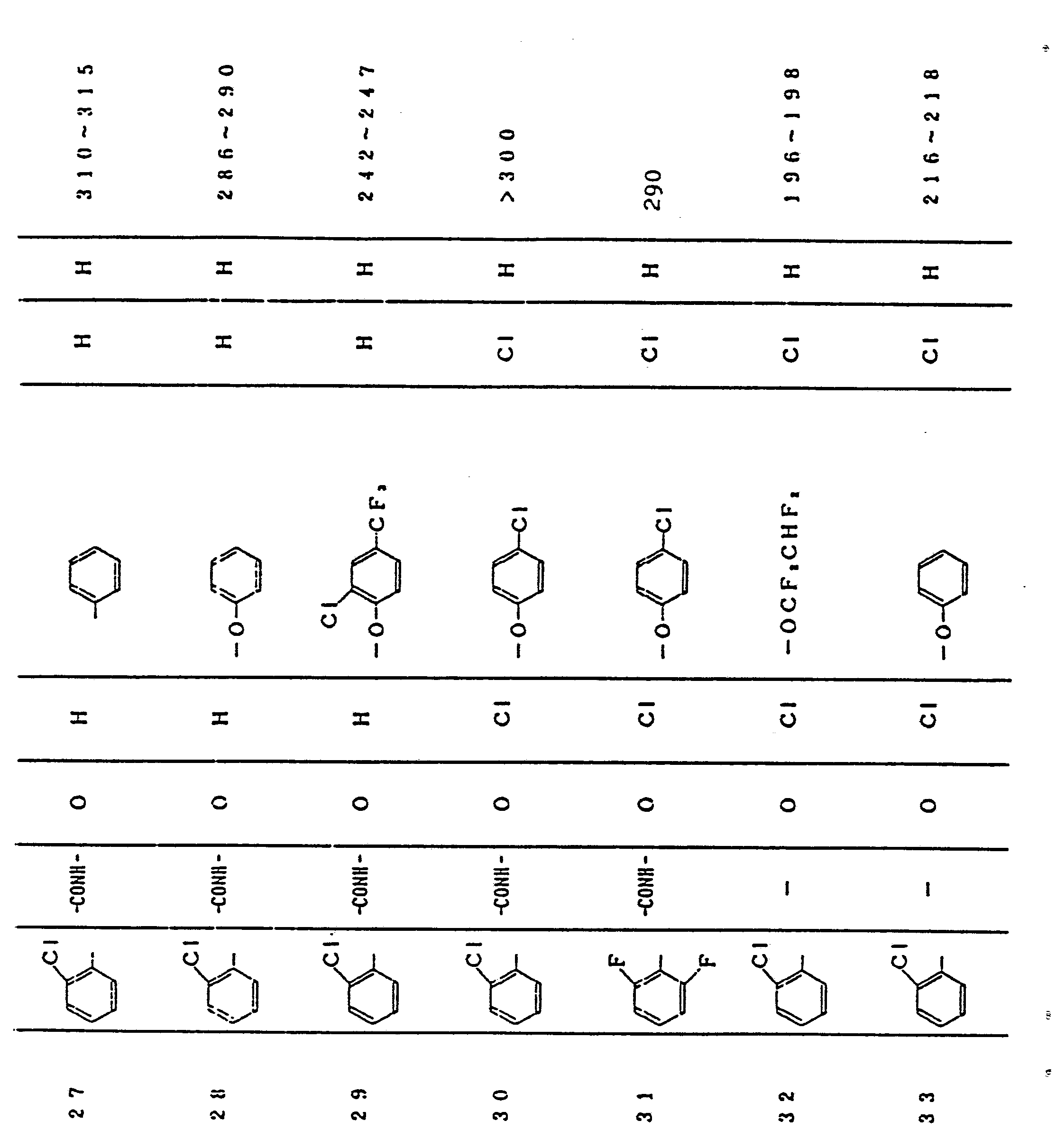

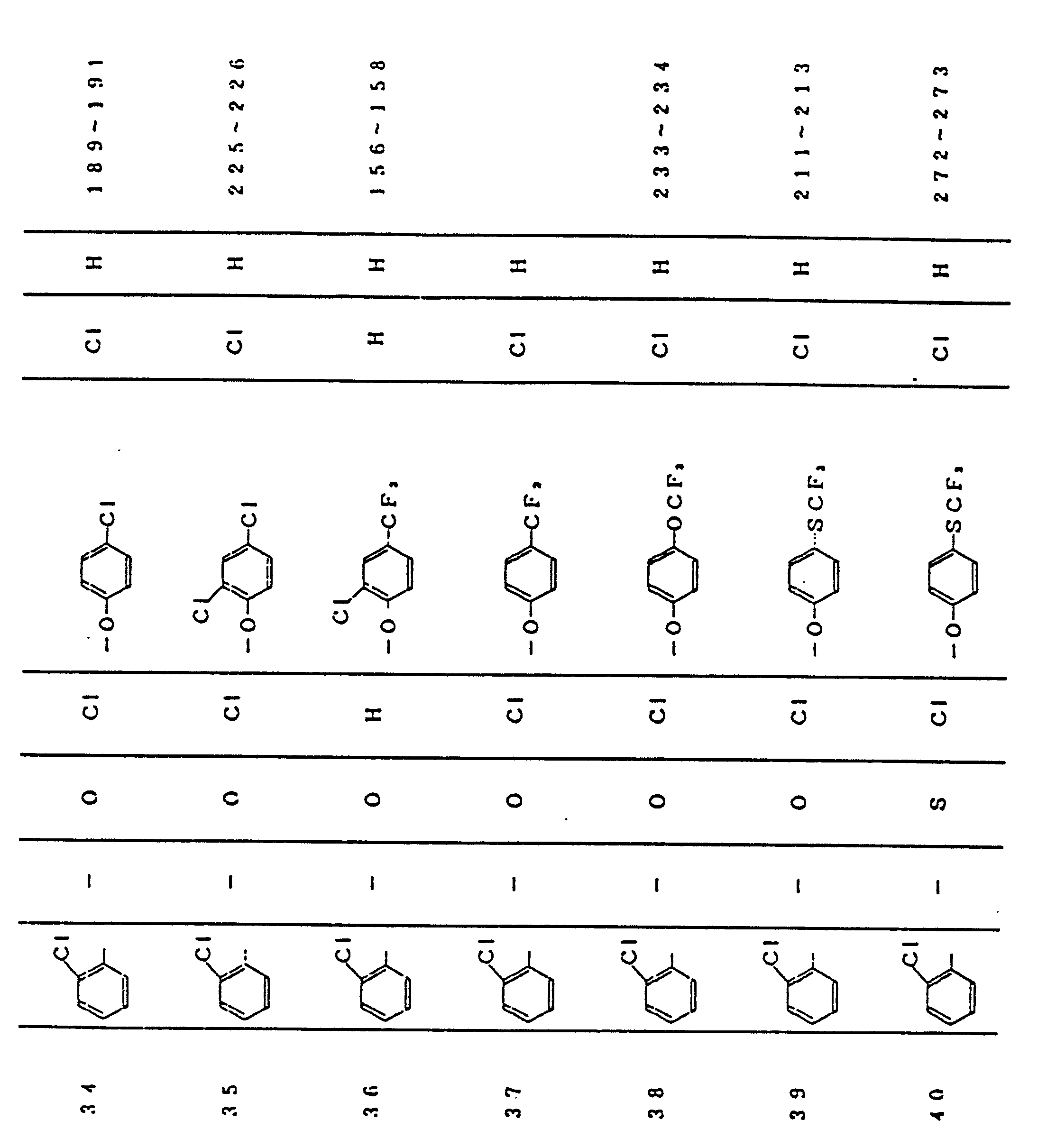

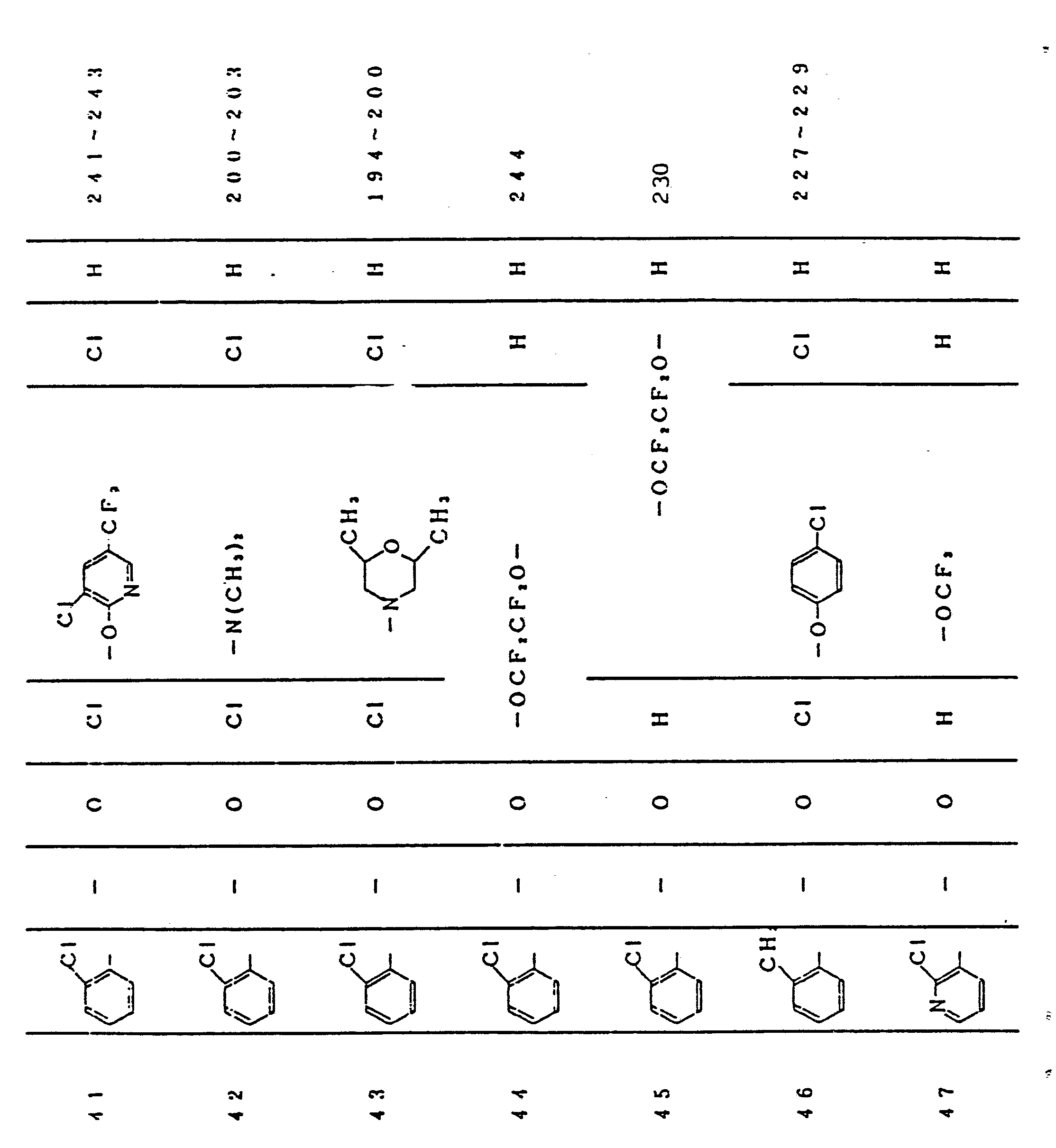

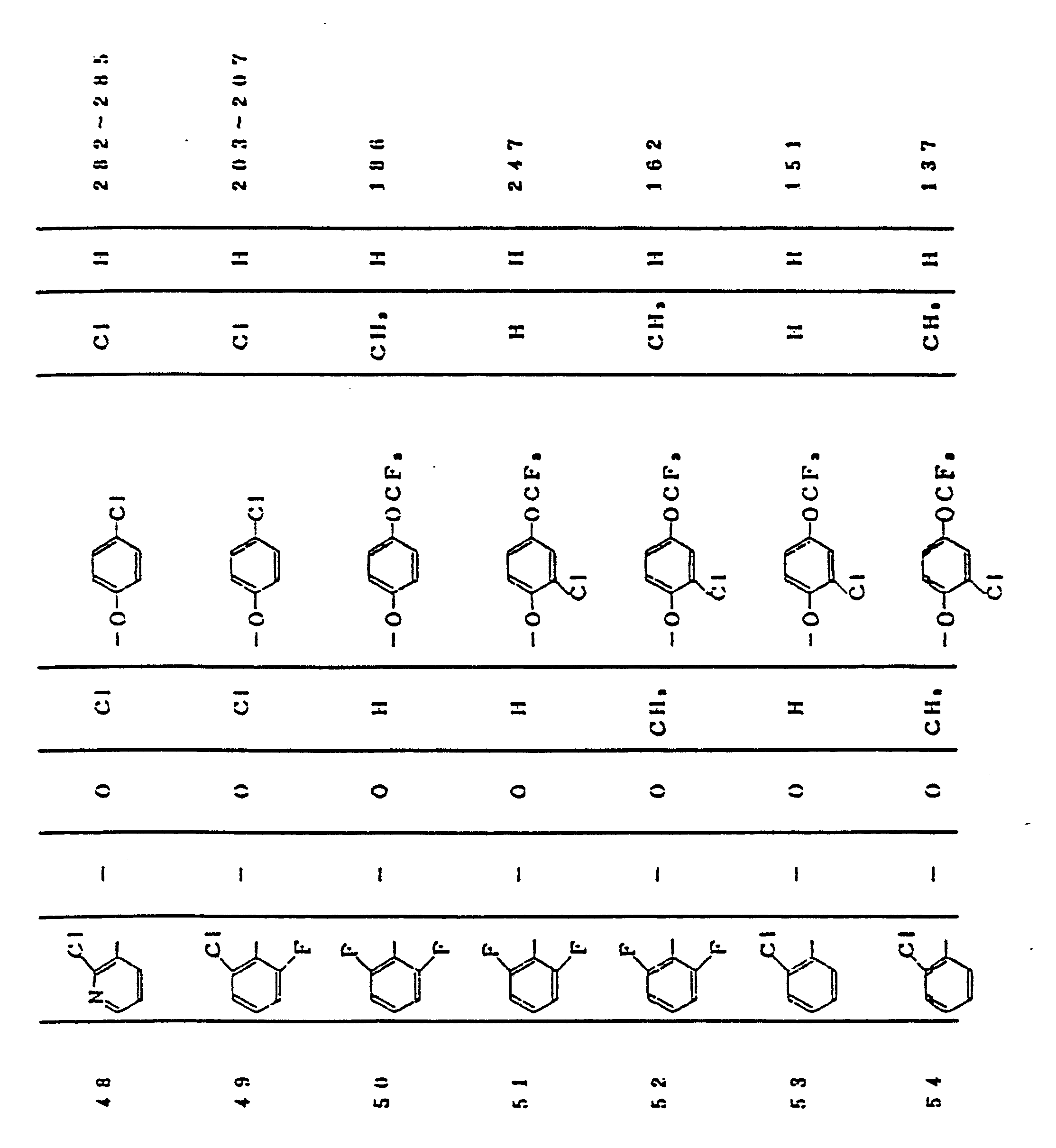

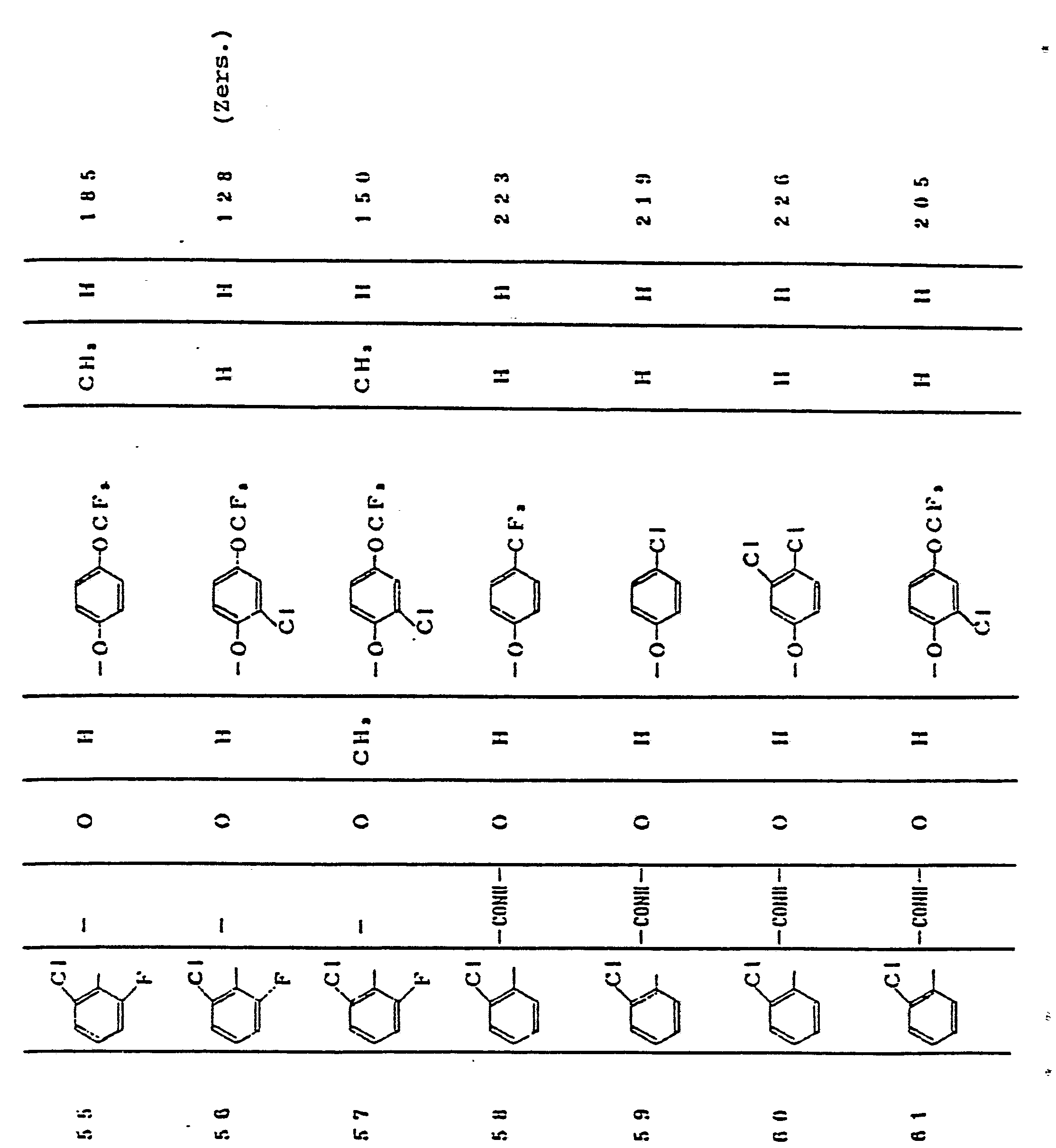

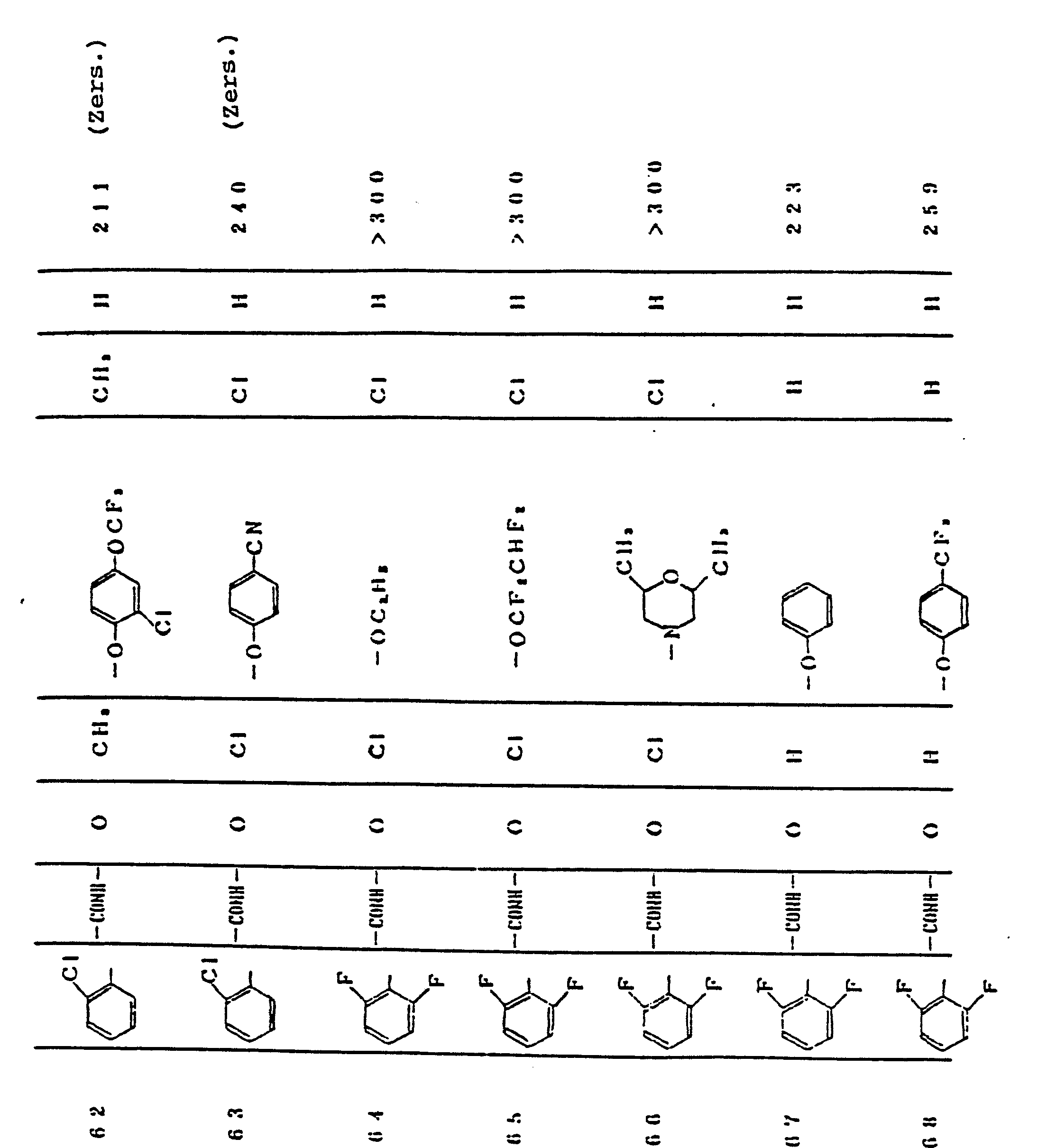

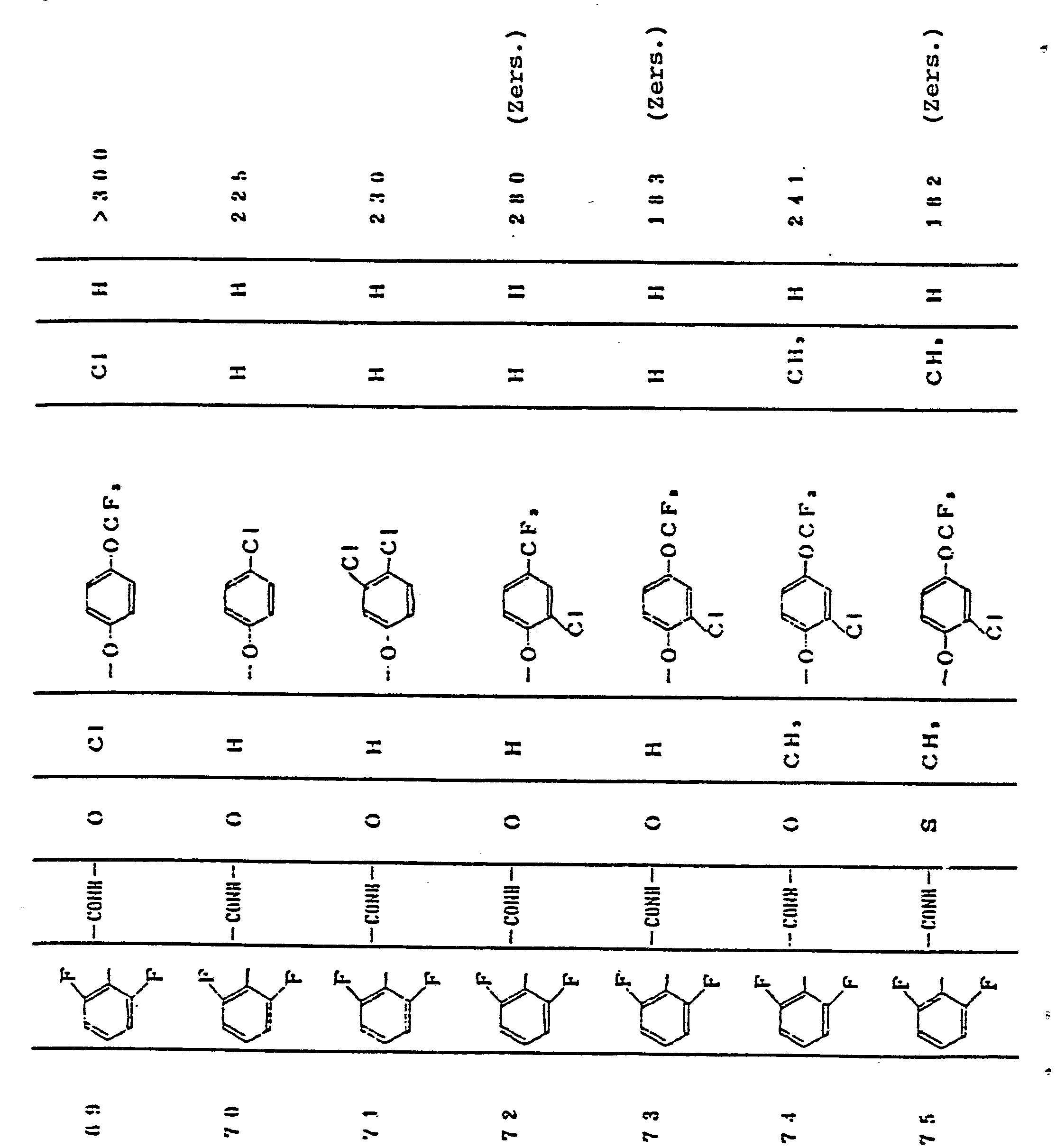

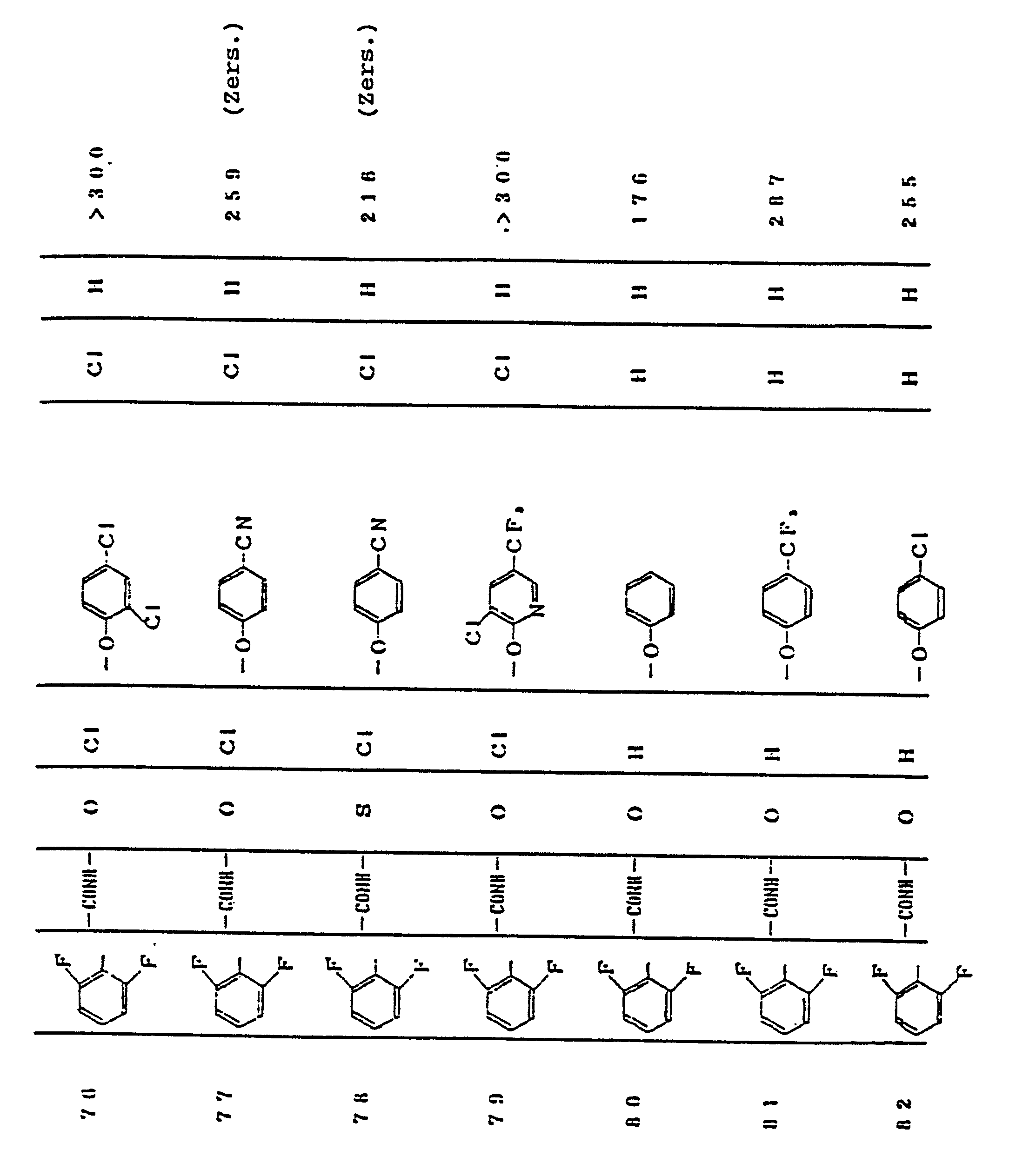

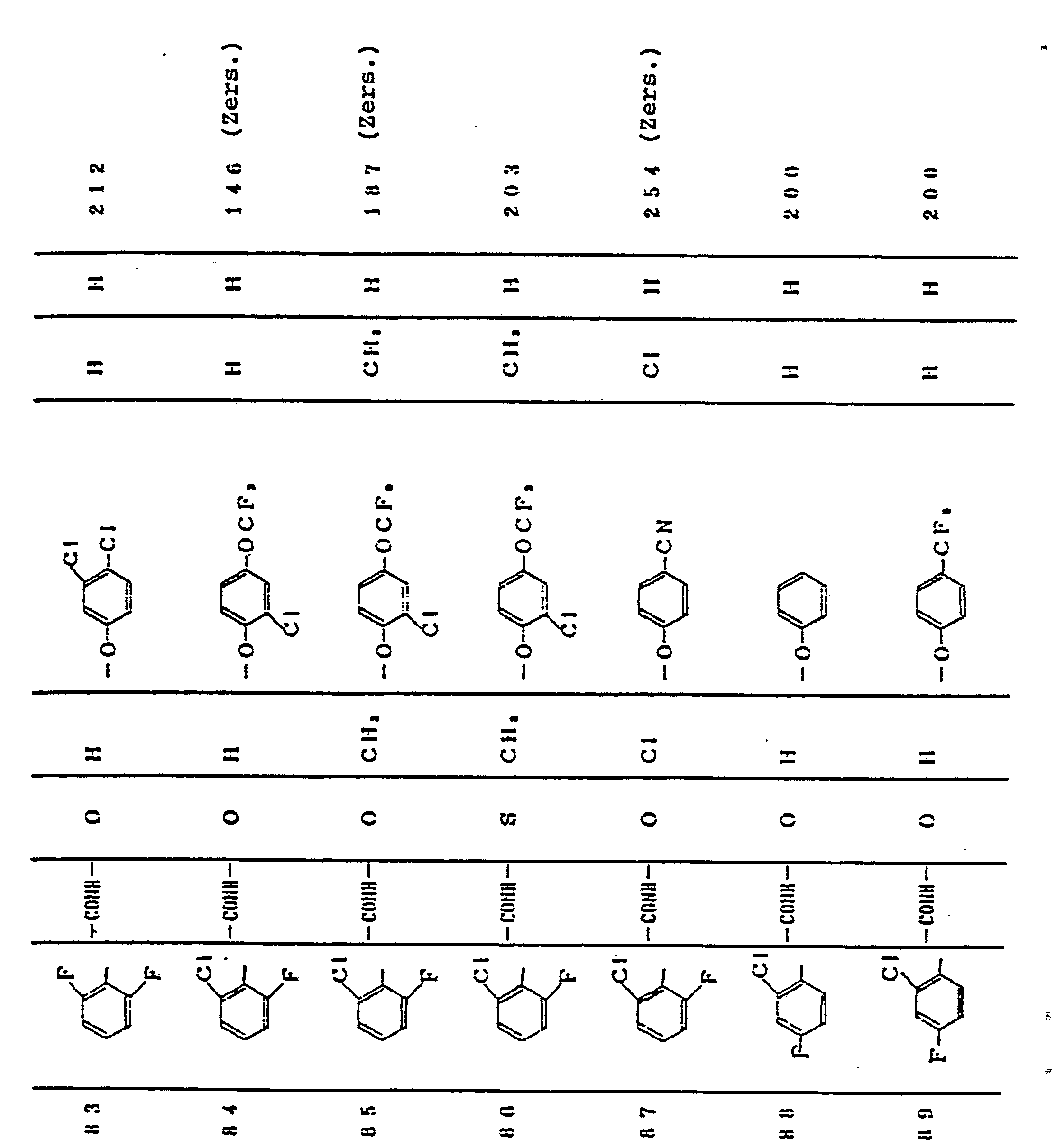

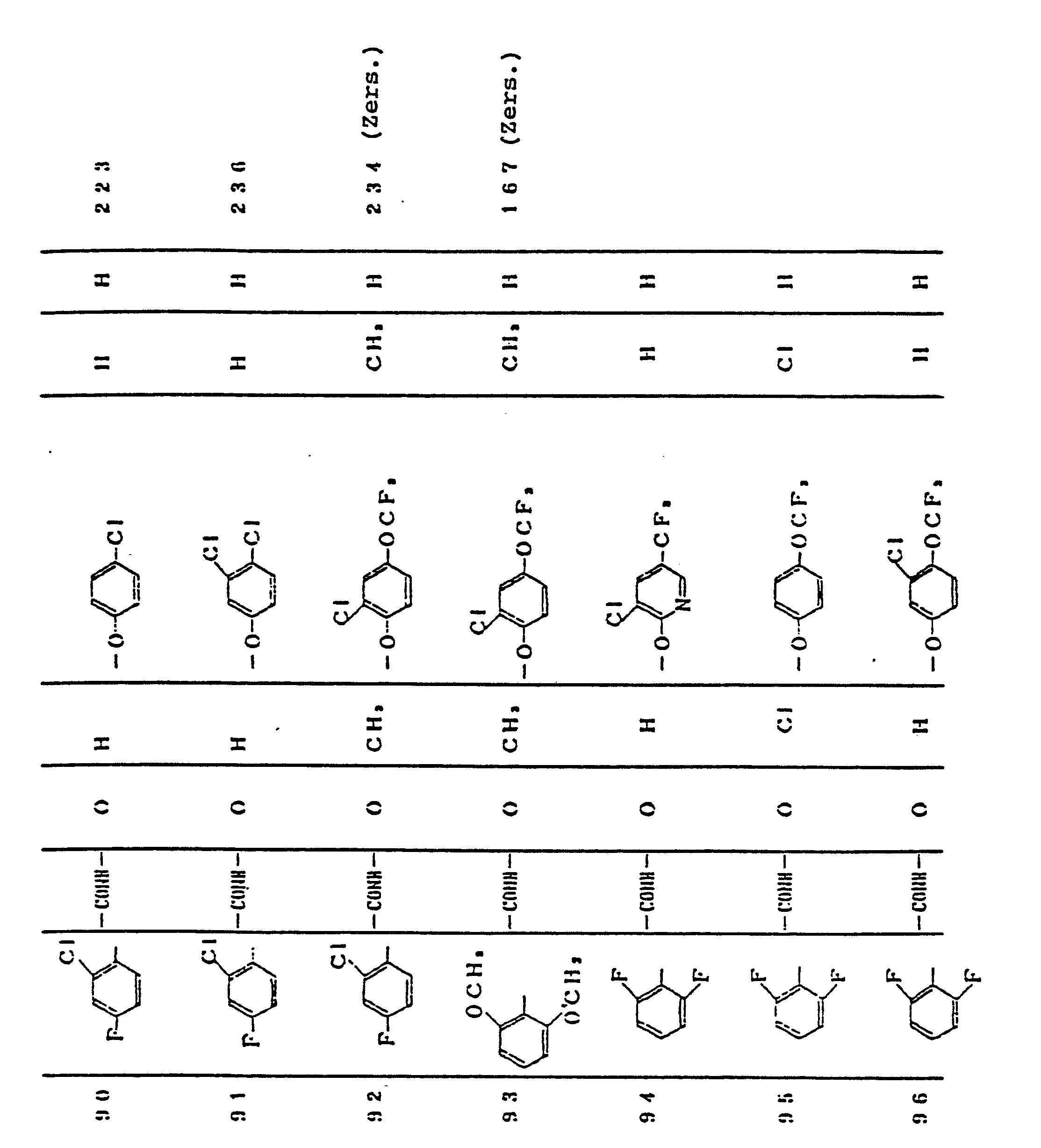

### Beispiel 5

Synthese eines Zwischenprodukts

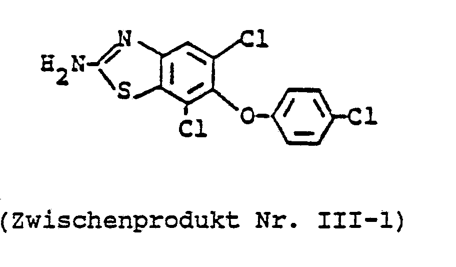

3,5-Dichloro-4-(4-chlorophenoxy)anilin (14,4 g) wurde in Essigsäure (250 ml) gelöst, und Kaliumthiocyanat (10 g) wurde der Lösung zugesetzt. Die Mischung wurde 20 min gerührt. Eine Lösung von Brom (8,4 g) in Essigsäure (30 ml) wurde tropfenweise bei 10 °C bis 20 °C hinzugefügt. Die Mischung wurde über Nacht bei Raumtemperatur gerührt. Danach wurde die Essigsäure unter vermindertem Druck abgedampft. Der Rückstand wurde in Wasser (100 ml) suspendiert und mit wäßrigem Ammoniak neutralisiert. Der resultierende Feststoff wurde durch Filtration gesammelt, mit Wasser gewaschen, getrocknet und aus Ethanol umkristallisiert, wonach das gewünschte 2-Amino-5,7-dichloro-6-(4-chlorophenoxy)benzothiazol (12 g) erhalten wurde; Schmp. 241-243 °C.

Tabelle 2 zeigt typische Beispiele für Zwischenprodukte, die nach der gleichen Methode wie in Beispiel 5 erhalten wurden.

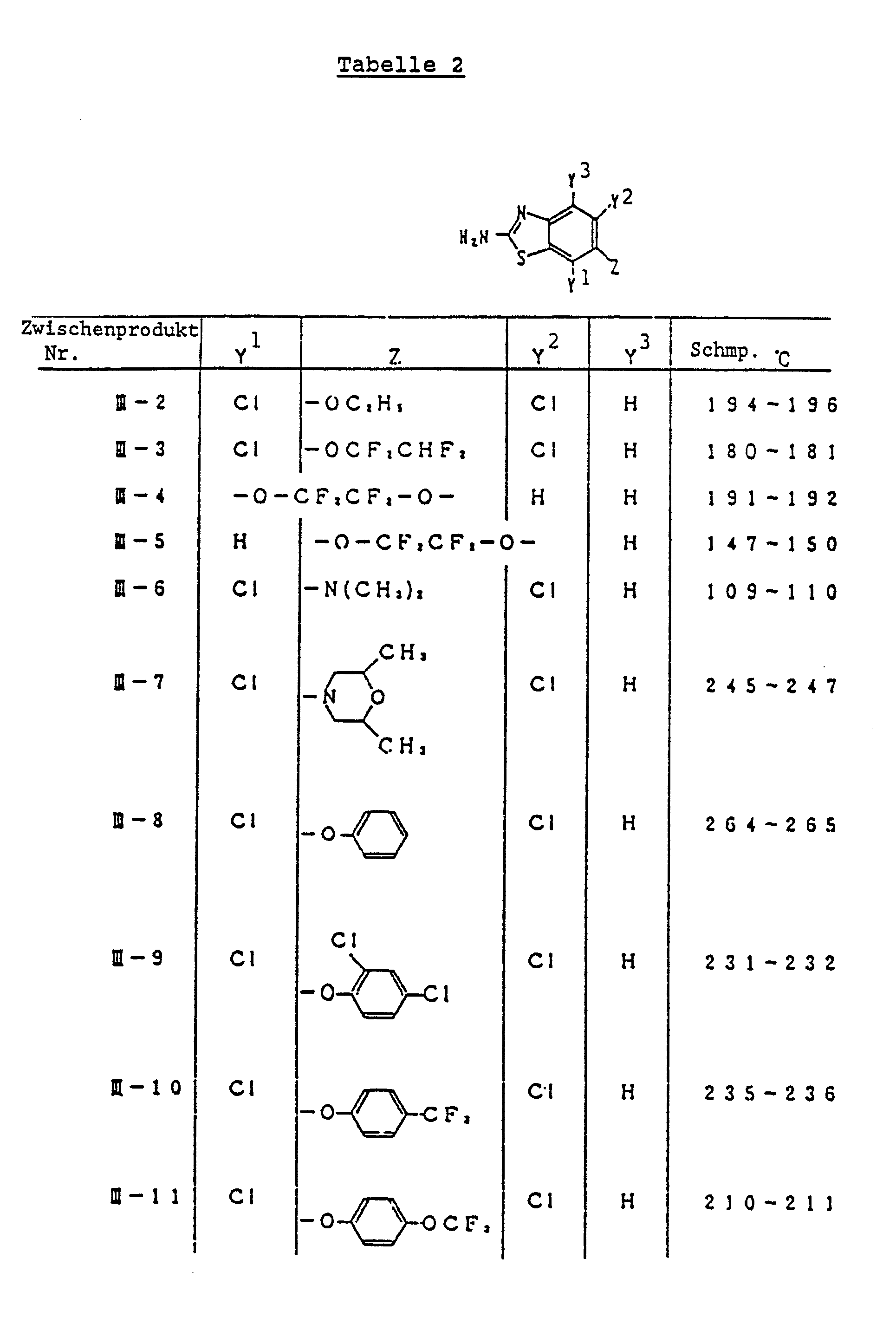

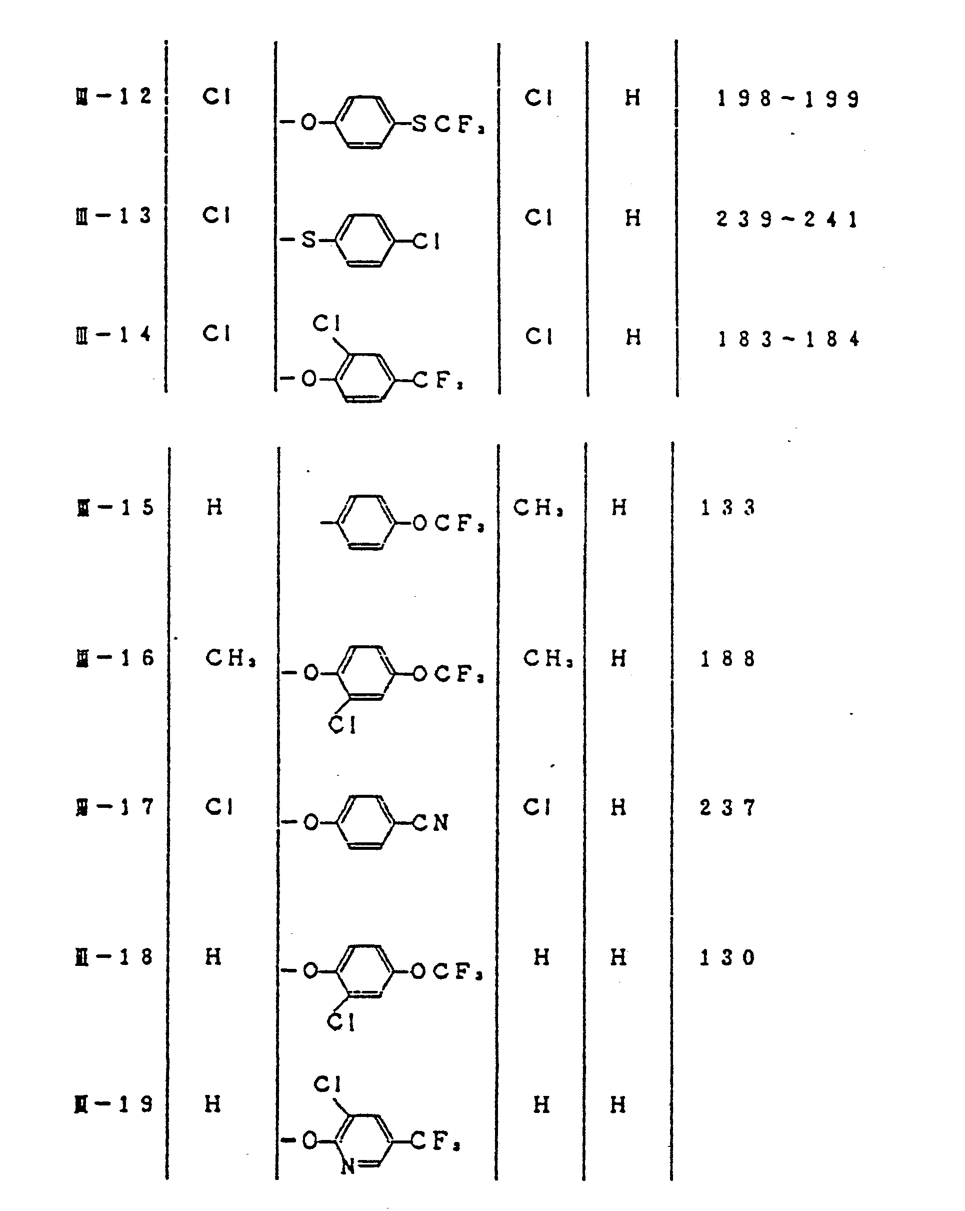

### Biologische Tests

Vergleichs-Verbindung E-1: 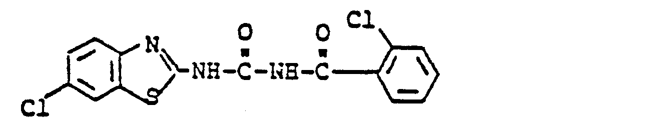 (die in der JP-OS 124 265/1978 beschriebene Verbindung);
Vergleichs-Verbindung G-1: 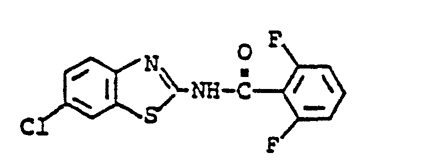 (die in J. Agric. Food Chem., Band 24, 1976, Seiten 1065-1068, beschriebene Verbindung).

### Beispiel 6

Test mit Larven von Spodoptera litura:
Herstellung einer Test-Chemikalie: Lösungsmittel: 3 Gew.-Teile Dimethylformamid
Emulgator: 1 Gew.-Teil Polyoxyethylenalkylphenylether

1 Gew.-Teil jeder aktiven Verbindung wurde mit dem Lösungsmittel und dem Emulgator in den oben angegebenen Mengen vermischt, und die Mischung wurde zur Herstellung einer Test-Chemikalie mit Wasser auf eine vorher festgesetzte Konzentration verdünnt.

Test-Verfahren:
Kohlblätter wurden in die wäßrige Verdünnung der aktiven Verbindung mit der vorbestimmten Konzentration getaucht und dann wieder entnommen. Die Chemikalie wurde an der Luft trocknen gelassen. Dann wurden die Kohlblätter in eine Petrischale von 9 cm Durchmesser gelegt. 5 Larven im 3. Stadium von Spodoptera litura wurden in die Petrischale eingesetzt, und die Petrischale wurde dann in einen Inkubator von 28 °C gestellt. Sieben Tage später wurde das Tötungsverhältnis berechnet.

Anhand der Einbeziehung typischer Beispiele sind die Ergebnisse in Tabelle 3 aufgeführt.

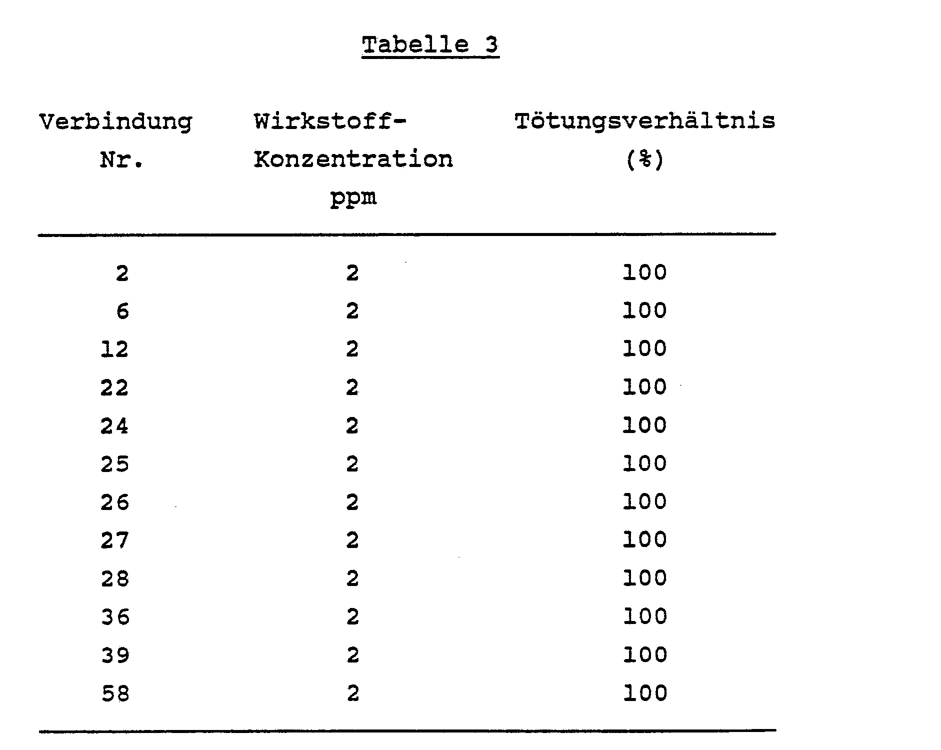

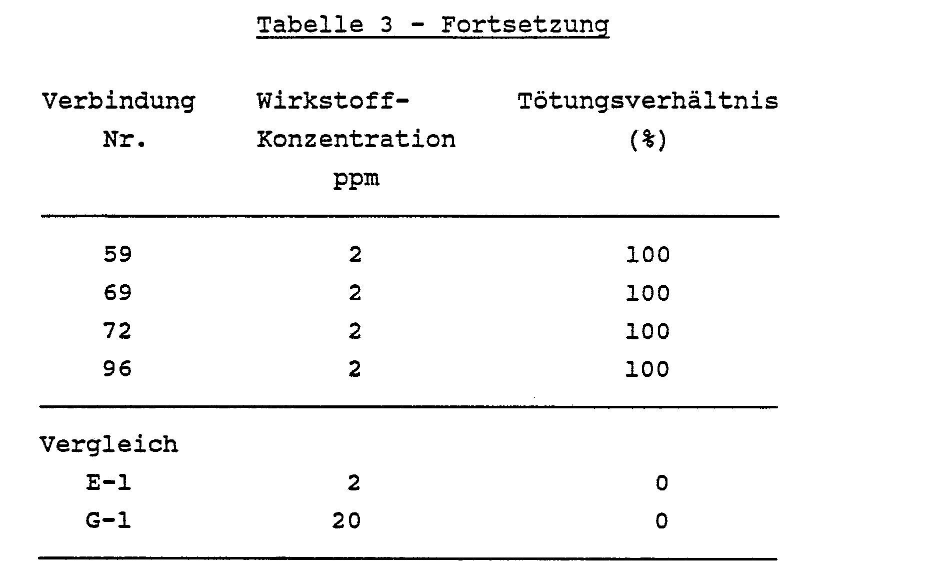

### Beispiel 7

Test mit Larven von Plutella maculipennis:
Kohlblätter wurden in die wäßrige Verdünnung jeder aktiven Verbindung mit der vorbestimmten Konzentration, die wie in Beispiel 6 hergestellt worden war, getaucht und dann wieder entnommen. Die Chemikalie wurde an der Luft trocknen gelassen. Dann wurden die Kohlblätter in eine Petrischale von 9 cm Durchmesser gelegt. 10 Larven im 2. Stadium von Plutella maculipennis wurden in die Petrischale eingesetzt, und die Petrischale wurde dann in einen Inkubator von 23 °C gestellt. Sieben Tage später wurde das Tötungsverhältnis berechnet.

Anhand der Einbeziehung typisher Beispiele sind die Ergebnisse in Tabelle 4 aufgeführt.

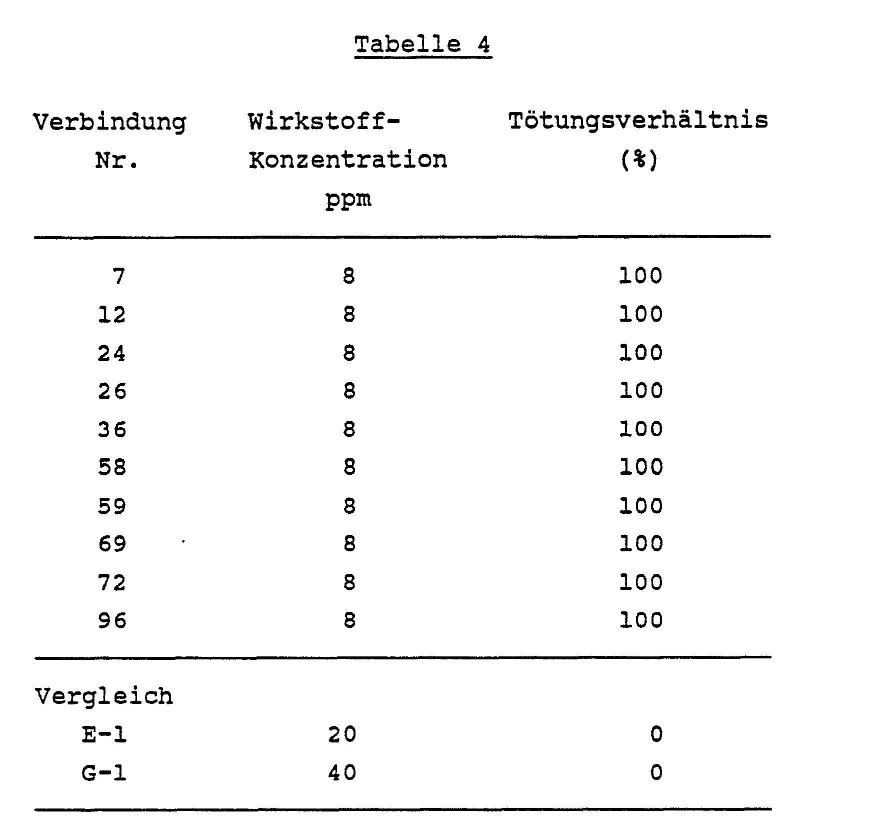

## Patentansprüche

1. Neue N-Benzothiazolylamide der Formel (I) 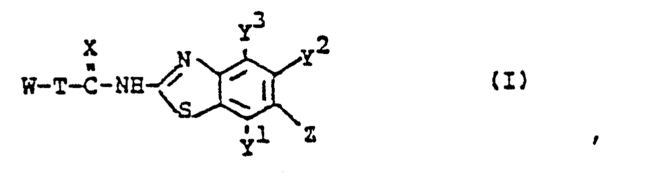 in der
X Sauerstoff oder Schwefel ist,
T eine Einfachbindung oder ←CONH-ist, worin der Pfeil "-" eine Bindung mit W bezeichnet, Y¹, Y² und Y³ gleich oder verschieden sind und H, Halogen oder Alkyl bezeichnen,
Z Halogen, Alkoxy, Halogenoalkoxy, Aralkyloxy, Alkenyloxy, Halogenoalkenyloxy, Alkinyloxy, 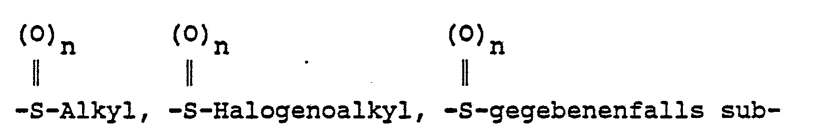 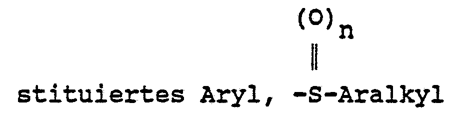 ist,
worin n jeweils 0, 1 oder 2 ist, oder
Z Aryl, gegebenenfalls substituiertes Aryloxy, -O-Q ist, worin Q eine gegebenenfalls substituierte 5-bis 6-gliedrige heterocyclische Gruppe ist, oder 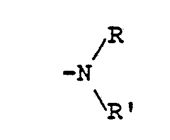
ist, worin R und R' gleich oder verschieden sind und Alkyl oder Alkenyl bezeichnen und R und R' zusammen mit dem N-Atom, an das sie gebunden sind, eine gegebenenfalls substituierte 5-bis 6-gliedrige heterocyclische Gruppe bilden können, die Hetero-Atome neben einem Stickstoff-Atom enthalten kann, oder
Z zusammen mit Y¹ oder Y² eine Tetrafluoroethylendioxy-Gruppe bilden kann und 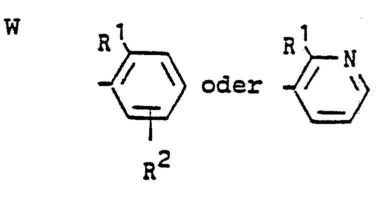
ist, worin R¹ Halogen, Alkyl oder Alkoxy ist und R² Wasserstoff, Halogen, Alkyl oder Alkoxy ist, mit der Maßgabe, daß
(a) in dem Fall, in dem T ←CONH-ist, einer oder zwei der Substituenten Y¹, Y², Y³ und Z Halogen ist und die übrigen von ihnen Wasserstoff sind und W 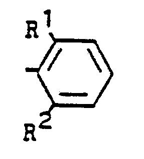
ist,
dann entweder R¹ oder R² Alkyl oder Alkoxy ist,
(b) in dem Fall, in dem T -CONH-ist, Y¹, Y² und Y³ Wasserstoff sind, Z Halogenoalkoxy oder 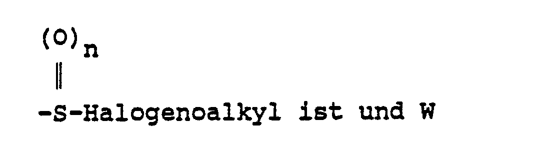 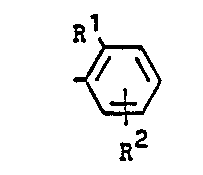 ist,
dann entweder R' oder R² Alkoxy ist.
(c) in dem Fall, in dem T eine Einfachbindung ist, Y¹, Y² und Y³ Wasserstoff sind, Z Halogenoalkoxy oder 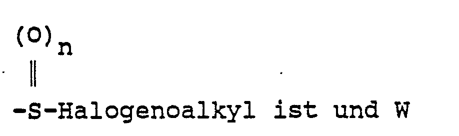 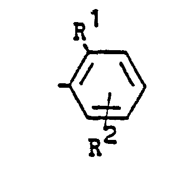 ist,
dann R' und R² Alkoxy sind, oder
(d) in dem Fall. in dem T eine Einfachbindung ist. Y¹. Y² und Y³ Wasserstoff sind, Z Halogen ist und W 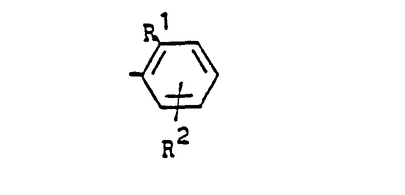
ist,
dann entweder R' oder R² Alkoxy ist.

2. Verbindungen der Formel (I) nach Anspruch 1, worin
X Sauerstoff oder Schwefel bezeichnet,
T eine Einfachbindung oder ←CONH-bezeichnet.
Y' und Y² gleich oder verschieden sind und Wasserstoff, Fluoro, Chloro, Bromo oder C₁-C₄-Alkyl bezeichnen,
Y³ Wasserstoff bezeichnet,
Z C₁-C₄-Alkoxy, C₁-C₄-Halogenoalkoxy, C₂-C₄-Halogenoalkenyloxy, C₆-C₁₀-Aryl, Phenoxy, das durch wenigstens einen Substituenten ausgewählt aus Fluoro, Chloro, Bromo, Trifluoromethyl, Trifluoromethoxy, Trifluoromethylthio und Cyano substituiert sein kann, bezeichnet, oder
Z Phenylthio, das durch die oben für Phenoxy angegebenen Substituenten substituiert sein kann, 3-Chloro-5-trifluoromethyl-2-pyridyloxy oder eine Gruppe der Formel 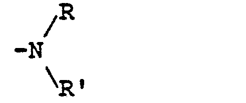 bezeichnet, worin R und R' identisch oder verschiedenartig sind und Cᵢ-C₄-Alkyl oder C₂-C₃-Alkenyl bezeichnen und R und R' zusammen mit dem N-Atom, an das sie gebunden sind, eine gegebenenfalls substituierte 6-gliedrige heterocyclische Gruppe bilden können, die ein Sauerstoff-Atom neben einem Stickstoff-Atom enthalten kann, und
Z zusammen mit Y¹ oder Y² eine Tetrafluoroethylendioxy-Gruppe bilden kann und
W 2-Chlorophenyl, 2-Chloro-6-fluorophenyl oder 2,6-Difluorophenyl bezeichnet, mit der Maßgabe, daß wenigstens einer der Substituenten Y¹ und Y² Fluoro, Chloro, Bromo oder C₁-C₄-Alkyl bezeichnet.

3. Verbindungen der Formel (I) nach Anspruch 1, worin
X Sauerstoff bezeichnet,
T eine Einfachbindung oder -CONH-bezeichnet,
Y¹ und Y² gleich oder verschieden sind und Wasserstoff oder Chloro bezeichnen,
Y³ Wasserstoff bezeichnet,
Z Ethoxy, 1,1,2,2-Tetrafluoroethoxy, Phenyl, Phenoxy, das durch wenigstens einen Substituenten ausgewählt aus Chloro, Trifluoromethyl und Trifluoromethoxy substituiert ist, bezeichnet, oder
Z Dimethylamino oder 2,6-Dimethylmorpholino ist und
W 2-Chlorophenyl oder 2,6-Difluorophenyl bezeichnet, mit der Maßgabe, daß wenigstens einer der Substituenten Y" und Y² Chloro bezeichnet.

4. Verbindungen nach den Ansprüchen 1 bis 3, nämlich
N-[5,7-Dichloro-6,-(1.1,2,2-tetrafluoroethoxy)benzothiazol-2-yl]-2.6-difluorobenzamid der folgenden Formel 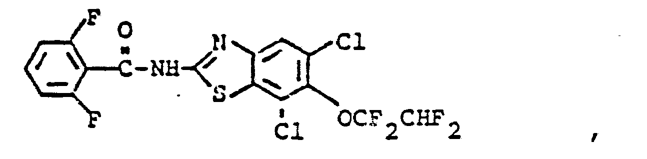
N-[5,7-Dichloro-6-(4-ch(orophenoxy)benzothiazol-2-yl]-2.6-difluorobenzamid der folgenden Formel 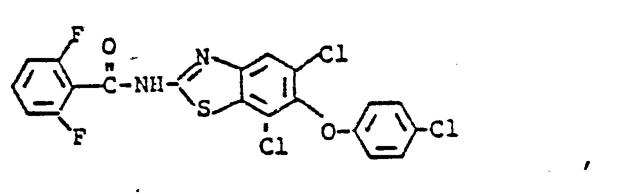
N-[5,7-Dichloro-6-(3-chloro-5-trifluoromefhyl-2-pyridyloxy)benzothiazol-2-yl]-2,6-difluorobenzamid der folgenden Formel 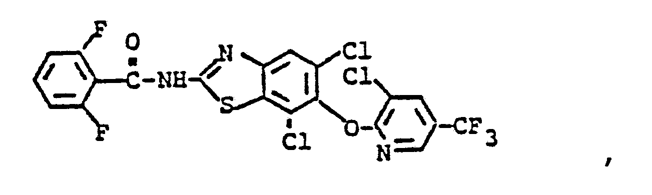
N-[5,7-Dichloro-6-(2-chloro-4-trifluoromethylphenoxy)benzothiazol-2-yl]-2,6-difluorobenzamid der folgenden Formel 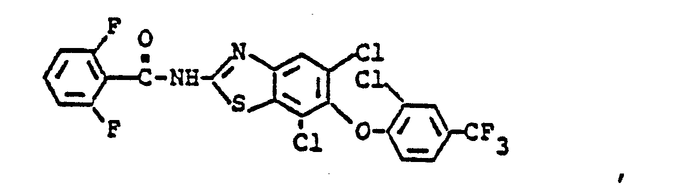
N-[5,7-Dichloro-6-(4-trifluoromethylphenoxy)benzothiazol-2-yl]-2,6-difluorobenzamid der folgenden Formel 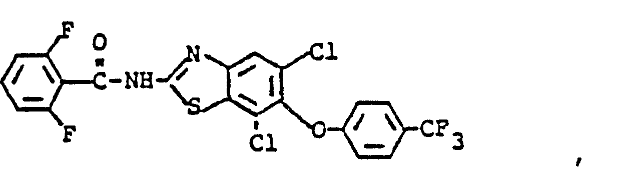
N-[5,7-Dichloro-6-(4-trifluoromethoxyphenoxy)benzothiazol-2-yl]-2,6-difluorobenzamid der folgenden Formel 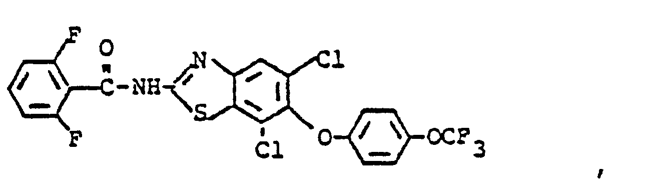
N-[5,7-Dichloro-6-(1,1,2,2-tetrafluoroethoxy)benzothiazol-2-yl]-2-chlorobenzamid der folgenden Formel 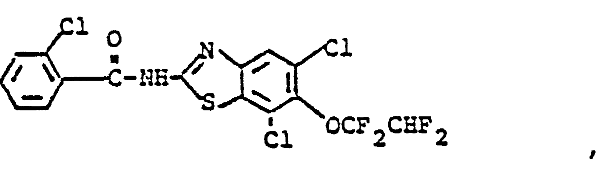
N-[5,7-Dichloro-6-(2,4-dichlorophenoxy)benzothiazol-2-yl]-2-chlorobenzamid der folgenden Formel 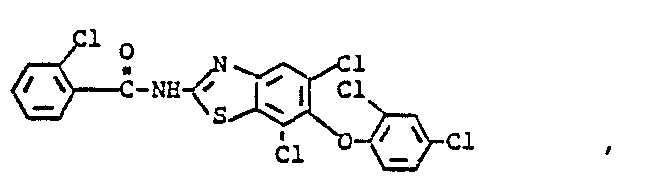
N-[5,7-Dichloro-6-(3-chloro-5-trifluoromethyl-2-pyridyloxy)benzothiazol-2-yl]-2-chlorobenzamid der folgenden Formel 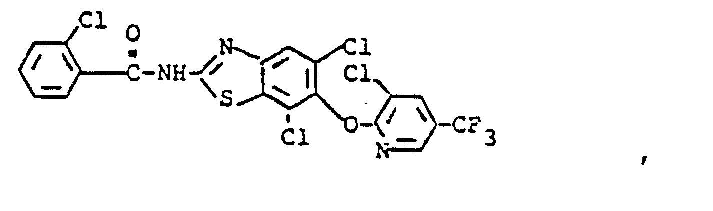 sowie die Verbindungen
1-(2,6-Difluorobenzoyl)-3-[6-(2-chloro-4-trifluoromethylphenoxy)benzothiazol-2-yl]harnstoff der folgenden Formel 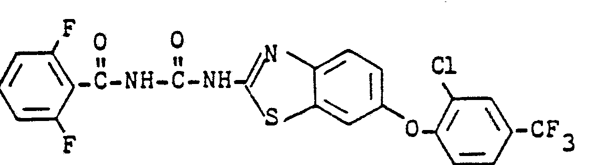
1-(2-Chiorobenzoyl)-3-[6-(2-chbro-4-trifiuoromethylphenoxy)benzothiazol-2-yl]harnstoff der folgenden Formel 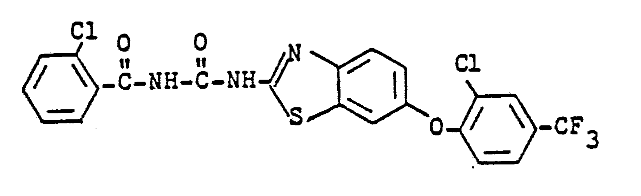
1-(2-Chlorobenzoyl)-3-[6-(4-chlorophenoxy)-5,7-dichlorobenzothiazol-2-yl]harnstoff der folgenden Formel 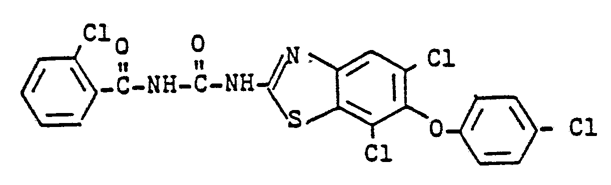
1-(2,6-Difluorobenzoyl)-3_{-[}6-(2-chloro-4-trifluoromethoxyphenoxy)benzothiazol-2-yl]harnstoff der folgenden Formel 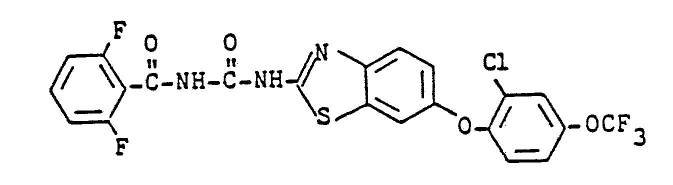
und 1-(2,6-Difluorobenzoyl)-3-[6-(4-trifluoromethoxyphenoxy)-5,7-dichlorobenzothiazol-2-yl]harnstoff der folgenden Formel

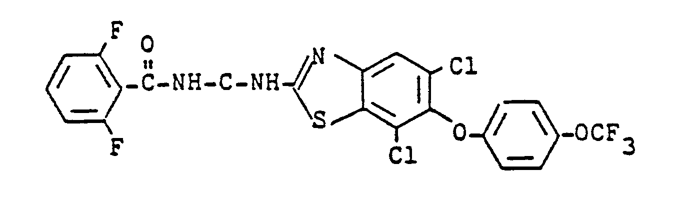

5. Verfahren zur Herstellung von N-Benzothiazolylamiden der Formel (I) 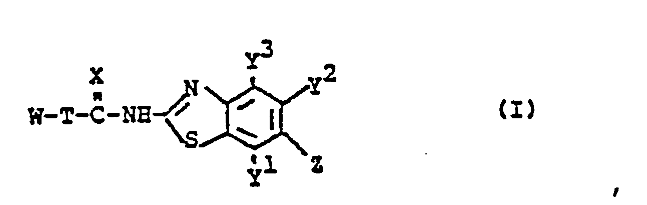
in der
X Sauerstoff oder Schwefel ist,
T eine Einfachbindung oder -CONH-ist, worin der Pfeil "←" eine Bindung mit W bezeichnet, Y¹, Y² und Y³ gleich oder verschieden sind und H, Halogen oder Alkyl bezeichnen,
Z Halogen, Alkoxy, Halogenoalkoxy, Aralkyloxy, Alkenyloxy, Halogenoalkenyloxy, Alkinyloxy, 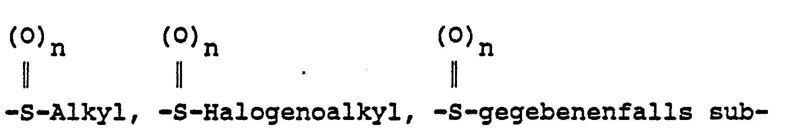 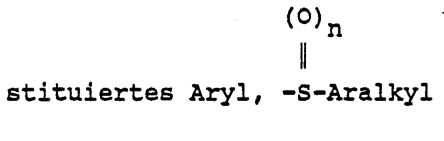 ist,
worin n jeweils 0, 1 oder 2 ist, oder
Z Aryl. gegebenenfalls substituiertes Aryloxy, -O-Q ist, worin Q eine gegebenenfalls substituierte 5-bis 6-gliedrige heterocyclische Gruppe ist, oder 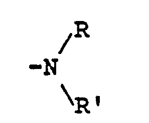 ist, worin R und R' gleich oder verschieden sind und Alkyl oder Alkenyl bezeichnen und R und R' zusammen mit dem N-Atom, an das sie gebunden sind, eine gegebenenfalls substituierte 5-bis 6-gliedrige heterocyclische Gruppe bilden können, die Hetero-Atome neben einem Stickstoff-Atom enthalten kann, oder
Z zusammen mit Y' oder Y² eine Tetrafluoroethylendioxy-Gruppe bilden kann und 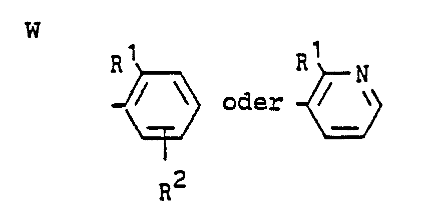 ist, worin R' Halogen, Alkyl oder Alkoxy ist und R² Wasserstoff, Halogen, Alkyl oder Alkoxy ist, mit der Maßgabe, daß
(a) in dem Fall, in dem T ←CONH-ist, einer oder zwei der Substituenten Y¹, Y², Y³ und Z Halogen ist und die übrigen von ihnen Wasserstoff sind und W 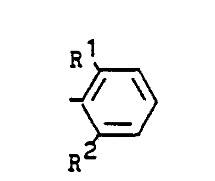 ist, dann entweder R¹ oder R² Alkyl oder Alkoxy ist,
(b) in dem Fall, in dem T -CONH-ist, Y¹, Y² und Y³ Wasserstoff sind, Z Halogenoalkoxy oder 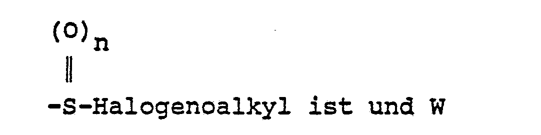 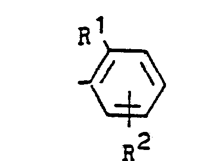 ist, dann entweder R' oder R² Alkoxy ist,
(c) in dem Fall, in dem T eine Einfachbindung ist, Y¹, Y² und Y³ Wasserstoff sind, Z Halogenoalkoxy oder 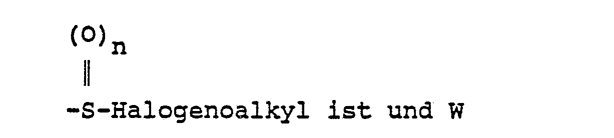 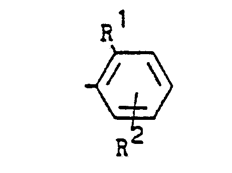 ist, dann R¹ und R² Alkoxy sind, oder
(d) in dem Fall, in dem T eine Einfachbindung ist, Y¹, Y² und Y³ Wasserstoff sind, Z Halogen ist und W 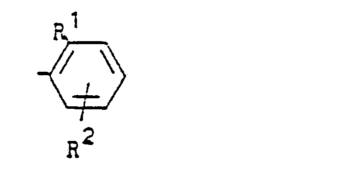 ist, dann entweder R¹ oder R² Alkoxy ist, dadurch gekennzeichnet, daß
a) in dem Fall, in dem X Sauerstoff ist und T eine Einfachbindung ist, Verbindungen der Formel (II) 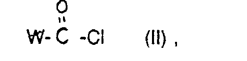 in der W die im Vorstehenden angegebenen Bedeutungen hat, mit Verbindungen der Formel 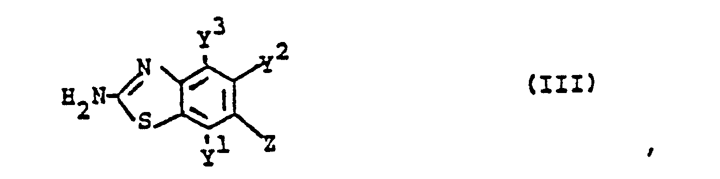 in der Y¹, Y², Y³ und Z die im Vorstehenden angegebenen Bedeutungen haben, in Gegenwart eines inerten Lösungsmittels und gegebenenfalls in Gegenwart einer Base umgesetzt werden, oder
b) in dem Fall, in dem X Schwefel ist und T eine Einfachbindung ist, Verbindungen der Formel (IV) 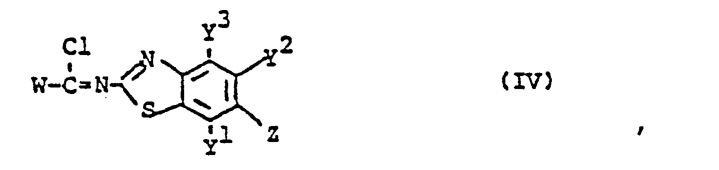 in der Y¹, Y², Y³, Z und W die im Vorstehenden angegebenen Bedeutungen haben, mit Hydrogensulfid in Gegenwart eines inerten Lösungsmittels und gegebenenfalls in Gegenwart einer Base umgesetzt werden. oder
c) in dem Fall, in dem T -CONH-ist, Verbindunqen der Formel (V) 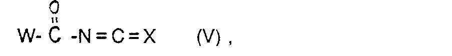 in der W und X die im Vorstehenden angegebenen Bedeutungen haben, mit den oben bezeichneten Verbindungen der Formel (III) in Gegenwart eines inerten Lösungsmittels umgesetzt werden.

6. Insektizide Mittel. dadurch gekennzeichnet, daß sie wenigstens ein N-Benzothiazolylamid der Formeln nach den Ansprüchen 1 bis 4 oder der Formel (I) nach Anspruch 5 enthalten.

7. Verfahren zur Bekämpfung schädlicher Insekten, dadurch gekennzeichnet, daß man N-Benzothiazolylamide der Formeln nach den Ansprüchen 1 bis 4 oder der Formel (I) nach Anspruch 5 auf die schädlichen Insekten und_{/}oder deren Lebensraum einwirken läßt.

8. Verwendung von N-Benzothiazolylamiden der Formeln nach den Ansprüchen 1 bis 4 oder der Formel (I) nach Anspruch 5 zur Bekämpfung schädlicher Insekten.

9. Verfahren zur Herstellung insektizider Mittel, dadurch gekennzeichnet, daß N-Benzothiazolylamide der Formeln nach den Ansprüchen 1 bis 4 oder der Formel (I) nach Anspruch 5 mit Streckmitteln und/oder grenzflächenaktiven Mitteln vermischt werden.

10. Neue Aminobenzothiazole der Formel (III') 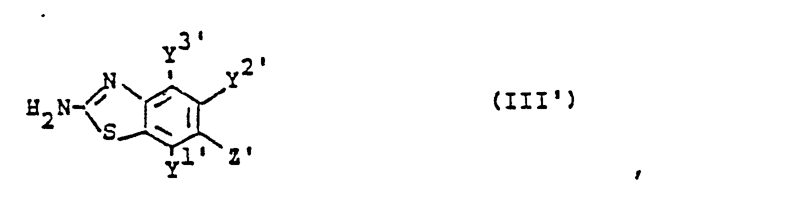 in der
Y^{1'} und Y^{2'} gleich oder verschieden sind und Wasserstoff. Halogen oder Alkyl bezeichnen.
Y3 Wasserstoff ist und
in dem Fall, in dem Y^{1'} und Y^{2'} Halogen oder Alkyl sind, dann
Z' Alkoxy, Halogenoalkoxy, Aryloxy, Arylthio, gegebenenfalls substituiertes Pyridyloxy oder einer Gruppe der Formel 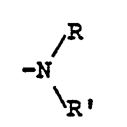 ist, worin R und R' jeweils Alkyl oder Alkenyl bezeichnen und R und R' zusammen mit dem N-Atom, an das sie gebunden sind, eine gegebenenfalls substituierte 5-bis 6-gliedrige heterocyclische Gruppe bilden können, die Hetero-Atome neben einem Stickstoff-Atom enthalten kann, oder in dem Fall, in dem entweder Y" oder Y^{2'} Wasserstoff ist, dann
Z' 2-Chloro-4-trifluoromethylphenoxy, 2-Chloro-4-trifluoromethoxyphenoxy, 4-Trifluoromethylphenoxy oder 4-Trifluoromethoxyphenoxy ist und
Z' zusammen mit Y^{1'} oder Y^{2'} eine Tetrafluoroethylendioxy-Gruppe bilden kann.
